# EUROPEAN PATENT SPECIFICATION

(11) **EP 3 354 283 B1**
(45) Date of publication and mention of the grant of the patent: **07.08.2019**
(21) Application number: 18160014.9
(22) Date of filing: 05.03.2018
(51) Int. Cl.: A61K 9/48, A61K 31/4045, A61K 47/38, A61K 9/00

(54) **PHARMACEUTICAL CAPSULE COMPOSITION COMPRISING SILODOSIN**
PHARMAZEUTISCHE KAPSELZUSAMMENSETZUNG MIT SILODOSIN
COMPOSITION DE CAPSULE PHARMACEUTIQUE COMPRENANT DE LA SILODOSINE

(30) Priority: 20.06.2017 IN 201711021629; 01.08.2017 EP 17184249
(43) Date of publication of application: 01.08.2018
(73) Proprietor: Alfred E. Tiefenbacher (GmbH & Co. KG), 22767 Hamburg (DE)
(72) Inventor: KALAMATA, Venkatasimhadri Naidu, 502 319 Telangana (IN); BODDU, Rajkumar, 502 319 Telangana (IN); RALLABANDI, Bala Ramesha Chary, 502 319 Telangana (IN); STAVER, Ruslan, 22607 Hamburg (DE); SCHLEHAHN, Hendrik, 23843 Travenbrueck (DE)
(74) Representative: Hertin und Partner Rechts- und Patentanwälte PartG mbB

(56) References cited:
- EP-A1- 1 574 215
- WO-A1-2013/061338

## Description

The invention relates to a pharmaceutical composition in the form of a capsule, comprising a powder mixture comprising silodosin. The invention also relates to the use of said formulation in the treatment of dysuria and/or benign prostatic hyperplasia and to a method for the manufacture of the composition comprising mixing the excipients and silodosin in a single powder mixture or blend and direct filling into a capsule.

### BACKGROUND OF THE INVENTION

Benign prostatic hyperplasia (BPH) is a non-malignant enlargement of the prostate due to cellular hyperplasia of both glandular and stromal elements. As the prostate increases in size it may exert pressure on the lumen of the prostatic urethra, resulting in a gradual obstruction to urine flow.

Silodosin is an α-adrenergic antagonist that has high selectivity for the α1A receptor. Silodosin is used for the treatment of dysuria (such as BPH), has selective suppressing activities on the contraction of urethra smooth muscles, and is an extremely useful compound as a medicament for treating dysuria without causing strong hypotensive activities or orthostatic hypotension.

Silodosin (1-(3-hydroxypropyl)-5-[(2*R*)-({2-[2-[2-(2,2,2-trifluoroethoxy)phenoxy]ethyl}amino) propyl]indoline-7-carboxamide) is an indoline derivative having the molecular formula C₂₅H₃₂F₃N₃O₄ and the following chemical structure:

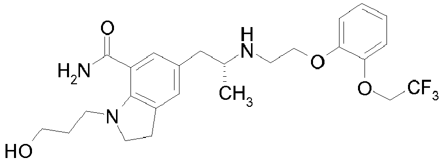

Silodosin is approved in the USA and EU for 4 mg twice daily dosing and 8 mg once daily dosing to treat symptoms associated with BPH. Silodosin is marketed under the tradenames Rapaflo (USA), Silodyx (EU), Urorec (EU), Urief (JP).

Despite its suitability for the treatment of symptoms associated with BPH, the pharmaceutical formulation of Silodosin proves challenging due to physical properties of the molecule.

Silodosin has been described as possessing incompatibilities with some pharmaceutical excipients and also yields degradation products. For example, a most popularly used filler, lactose, leads to difficulties when used in formulating tablets of silodosin. The resulting tablets exhibit undesirable dissolution properties and unsatisfactory hardness. Moreover, silodosin has potent adhesive properties, and in the case of preparing a tablet or capsule the use of a lubricant is typically required. However, the addition of lubricants leads to further difficulties with respect to extending dissolution time.

The prior art teaches that, in cases where formulations are prepared by a dry process, electrostatic charges are generated by physical irritations caused through processes such as pulverization, agitation, blending, granulation and the like, which in turn cause a decrease in fluidity of pulverized, blended or granulated materials, worsened handling properties, and ultimately a decrease in precision for content uniformity of the active ingredient (EP 1574215). In light of these difficulties, improved or alternative means for preparing pharmaceutical formulations of silodosin are required. Various attempts at providing stable compositions of silodosin have been described in the art.

WO 2013/061338 discloses stable formulations comprising silodosin, wherein granules are filled into capsules.

EP 1574215 discloses silodosin granules obtained via wet granulation, and teaches that formulations prepared through a wet granulating process, in particular by adding a lubricant and surfactant post-granulation, have acceptable content uniformity and dissolution properties. WO 2014/006635 discloses surfactant-free dry silodosin blends employing sodium stearyl fumarate as lubricant.

WO 2015/152680 discloses a composition obtained via wet granulation using alkaline inorganic salts, e.g. calcium carbonate, as stabilizer.

WO 2012/010669 discloses the use of a basic copolymer as a stabilizer in a silodosin formulation.

WO 2012/000926 discloses the formation of a silodosin-cyclodextrin inclusion complex in order to stabilize the granulated silodosin formulation.

WO 2012/147107 A2 and WO 2017/055664 A1 disclose methods for the production of silodosin and silodosin salts, respectively, in addition to pharmaceutical compositions comprising silodosin using low levels of PGS as a disintegrant.

The prior art teaches that additional stabilizers or complicated wet granulation methodologies are necessary in order to obtain suitable silodosin formulations. In light of the silodosin formulations described in the art, a need exists for providing stable silodosin formulations with acceptable content uniformity, stability and dissolution properties, without the need to employ the complicated multi-step wet granulation methods or additional stabilizing compounds, as have been suggested previously.

### SUMMARY OF THE INVENTION

In light of the prior art the technical problem underlying the invention was the provision of improved or alternative means for pharmaceutical compositions comprising silodosin that do not exhibit the disadvantages of the prior art.

This problem is solved by the features of the independent claims. Preferred embodiments of the present invention are provided by the dependent claims.

Therefore, the invention relates to a pharmaceutical composition according to the claims in the form of a capsule, comprising a powder mixture that comprises a) silodosin, b) a surfactant, c) a lubricant, and d) a disintegrant in an amount of 16% or more based on the total weight of all components of the mixture, and optionally e) a diluent.

In another embodiment, the invention relates to a pharmaceutical composition according to the claims in the form of a capsule, comprising a powder mixture that comprises a) silodosin, b) a surfactant, c) a lubricant, and d) low-substituted hydroxypropyl cellulose (L-HPC) as a disintegrant in an amount of 16% or more based on the total weight of all components of the mixture, and optionally e) a diluent.

In another embodiment, the invention relates to a pharmaceutical composition according to the claims in the form of a capsule, comprising a powder mixture that comprises a) silodosin, b) a surfactant, c) a lubricant, and d) a disintegrant in an amount of 25% or more based on the total weight of all components of the mixture, and optionally e) a diluent.

The invention is therefore related to silodosin-containing capsules according to the claims comprising a powder mixture in which an uncommonly large amount of disintegrant is employed. In particular, the use of low-substituted hydroxypropyl cellulose (L-HPC) as a disintegrant in an amount of 16% or more based on the total weight of all components of the mixture leads to excellent stability of the silodosin in the compositions of the present invention. High levels of other disintegrants may also lead to unexpectedly good stability of silodosin in the capsules.

To date, the prior art teaches that due to difficulties in handling, excipient compatibility and dissolution, effective formulations of silodosin must be prepared by granulation, in particular wet granulation, and subsequent extra-granular application of a lubricant and surfactant. The prior art is therefore plagued by complex and relatively cost-intensive requirements for manufacture of silodosin granules and capsules.

The pharmaceutical composition described herein is characterised in that said composition is prepared by dry mixing or blending the silodosin, disintegrant, surfactant, lubricant, and optional diluent, in a single powder mixture or blend, followed by direct filling into a capsule.

The most simple and cost-efficient process for capsule manufacturing relates to direct blending, producing a powder mixture, in place of granulation, hot melt extrusion, or spray drying. The present invention therefore enables simpler, less complicated and more cost-effective methods for manufacture of silodosin capsules that are comparable and improved with respect to stability and content uniformity over the granules of the prior art.

The method of manufacture according to the claims described herein, namely preparation by dry mixing or blending the silodosin, disintegrant, surfactant, lubricant, and optional diluent, in a single powder mixture or blend, followed by direct filling into a capsule, leads to inherent structural differences compared to those methods described in the prior art. The direct mixing of a blend and filing into a capsule can clearly be distinguished from the capsules comprising granules employed by the prior art.

The compositions of the present invention are characterised by unexpectedly good 6-month stability compared to formulations obtained via wet granulation (Urorec). The present invention also enables a simplified method of manufacture (no granulation) and reduced risk of impurities/instability due to avoiding solubilizing components and subsequent granulation or spray drying.

It was unexpectedly identified by the inventors that the presence of a disintegrant, low-substituted hydroxypropyl cellulose (L-HPC), in amounts higher than the 5-10% usually applied in solid pharmaceutical forms, leads to stable and amenable pharmaceutical compositions of silodosin.

According to the prior art, formulations prepared using dry processes, such as granulation or blending, generate electrostatic charges and a decrease in fluidity of the material, leading to difficulties in capsule filling and uniformity of fill volume. The formulations of the present invention, preferably defined by the combined incorporation of surfactants and lubricants, in combination with larger relative amounts of disintegrant lead to unexpectedly good fluidity and reduced adhesive properties, thereby enabling acceptable handling and uniformity of fill volume.

Furthermore, the pharmaceutical compositions described herein show surprisingly good polymorphic stability of the particular form of silodosin employed.

It appears therefore that the employment of relatively large amounts of disintegrant in the powder blend enable suitable handling of the powder blends, good content uniformity, good API stability (according to both chemical/impurity and polymorphic analyses) and good dissolution of the API. This combination of improved properties over the prior art would not have been expected by a skilled person in light of the apparent requirement of wet granulation or spray drying previously described in the art.

In one embodiment of the invention the pharmaceutical composition described herein is characterised in that the composition is in the form of a capsule, comprising a powder mixture that comprises a) silodosin, b) a surfactant, c) a lubricant, and d) a disintegrant, preferably low-substituted hydroxypropyl cellulose (L-HPC), in an amount of 16% or more based on the total weight of all components of the mixture, and optionally e) a diluent, wherein the diluent is selected from D-mannitol, sorbitol, and/or xylitol.

In one embodiment of the invention the pharmaceutical composition described herein is characterised in that the disintegrant is selected from low-substituted hydroxypropyl cellulose (L-HPC), pregelatinized starch (PGS), crospovidone, and/or microcrystalline cellulose (MCC).

In preferred embodiments the invention is characterised in that the disintegrant comprises or consists of low-substituted hydroxypropyl cellulose (L-HPC), although embodiments of the invention may employ pregelatinized starch (PGS), crospovidone, and/or microcrystalline cellulose (MCC), as alternatives or in addition to L-HPC.

In one embodiment of the invention the disintegrant is present in an amount of 16% or more based on the total weight of all components of the mixture. In one embodiment the 16% or more disintegrant can be specified alternatively as 16 wt% or more water-insoluble filler/binder/disintegrant. In some embodiments, a soluble diluent may also be employed.

A disintegrant is typically an agent used in the preparation of solid pharmaceutical formulations which causes them to disintegrate and release their medicinal substances on contact with moisture. However, the disintegrant may also be considered as a filler and/or binder, depending on the properties of the particular excipient employed as a disintegrant. A binder is typically a substance used to create a desired consistency in a formulation, whereby a filler is typically considered as a bulking agent to increase the mass of the formulation. Accordingly, the disintegrant exhibits properties of disintegration upon contact with aqueous environments, and is preferably a non-soluble disintegrant, but may also show properties associated with a filler or binder. PGS and L-HPC are generally considered as disintegrants, but either can also be considered as a water-insoluble filler. PGS and L-HPC may also have binder-like properties. With regards to MCC, besides being an excellent filler, microcrystalline cellulose also serves as a disintegrant (Jones, T. M.: The influence of physical characteristics of excipients on the design and preparation of tablets and capsules. Pharm. Ind. 39, 469-476, 1977).

In one embodiment of the invention the surfactant is sodium lauryl sulfate (SLS) and/or the lubricant is magnesium stearate. These excipients, typically functioning as a surfactant and lubricant, respectively, are preferred for their compatibility with silodosin either in combination or alone, or combined with other surfactants or lubricants. In one embodiment the lubricant is sodium stearyl fumarate.

In one embodiment of the invention the diluent is D-mannitol. In one embodiment of the invention, the diluent is D-mannitol and/or sorbitol, the disintegrant is L-HPC, the surfactant is SLS and the lubricant is magnesium stearate. This combination of excipients is one preferred embodiment and enables the beneficial stability, dissolution and handling as described herein.

In one embodiment of the invention, the disintegrant, low-substituted hydroxypropyl cellulose (L-HPC), is present in an amount of 16-96 wt%, preferably 18-50 wt%, more preferably 20-46%, based on the total weight of all components of the mixture. The amount of disintegrant (preferably either L-HPC and/or PGS) may therefore in some embodiments be 16% or more, or 17%, 18%, 19%, 20%, 21%, 22%, 23%, 24%, 25%, 26%, 27%, 28%, 29%, 30% or above, preferably a value as disclosed in the embodiments below. This unexpectedly large relative content of disintegrant, preferably in a powder blend directly filled into capsules, enables the beneficial stability, dissolution and handling as described herein.

In one embodiment of the invention, the pharmaceutical formulation according to the claims is characterized in that
a) the silodosin is present in an amount of 1-4 wt%, preferably 2-4 wt%,
b) the surfactant is present in an amount of 0.1-5 wt%, preferably 0.5-2 wt%,
c) the lubricant is present in an amount of 0.1-5 wt%, preferably 0.5-2 wt%,
d) the disintegrant is present in an amount 16-96 wt%, preferably 18-50 wt% and
e) the diluent is optionally present in an amount of 10-80 wt%, preferably 40-80 wt%, based on the total weight of all components of the mixture.

This unexpectedly large relative content of disintegrant, preferably in a powder blend directly filled into capsules, and in combination with the additional excipients disclosed above, enables surprisingly beneficial API stability, dissolution and blend handling as described herein.

In one embodiment of the invention, the diluent is D-mannitol and the disintegrant is L-HPC, and wherein the ratio of D-mannitol to L-HPC in the mixture is from 6:1 to 1:1, preferably from 4:1 to 1:1.

In one embodiment of the invention, the pharmaceutical formulation according to the claims is characterized in that
a) the silodosin is present in an amount of 1-4 wt%, preferably 2-4 wt%,
b) the surfactant is SLS and is present in an amount of 0.1-5 wt%, preferably 0.5-2 wt%,
c) the lubricant is magnesium stearate and is present in an amount of 0.1-5 wt%, preferably 0.5-2 wt%,
d) the disintegrant is L-HPC and is present in an amount 16-50 wt%, and
e) the diluent is D-mannitol and is present in an amount of 40-80 wt%, based on the total weight of all components of the mixture.

The formulation described above relates preferably to the specific formulations SIL/F1, SIL/F2 and SIL/F3. As demonstrated below, these formulations enable surprisingly beneficial API stability, dissolution and blend handling as described herein.

In further embodiments of the invention the formulation relates preferably to SIL/F1, SIL/F2 and SIL/F3, or alternative formulations based closely on SIL/F1, SIL/F2 and SIL/F3, as demonstrated below.

| | **Ingredients** | **wt%** |
|---|---|---|
| 1. | Silodosin | 1.0, 1.5, 2.0, 2.3, 2.5, 3.0, 3.5 or 4.0, or a value between the highest and lowest of the series |
| 2. | Diluent, preferably Mannitol | 40, 45, 50, 55, 60, 65, 70, 75 or 80, or a value between the highest and lowest of the series |
| 3. | Disintegrant, Low-substituted Hydroxypropylcellulose | 16, 20, 25, 30, 35, 40, 45 or 50, or a value between the highest and lowest of the series |
| 4. | Surfactant, preferably Sodium Lauryl Sulphate | 0.1, 0.5, 0.75, 1.0, 1.25, 1.5, 2, 2.5, 3, 4, or 5, or a value between the highest and lowest of the series |
| 5. | Lubricant, preferably Magnesium Stearate | 0.1, 0.5, 0.75, 1.0, 1.25, 1.5, 2, 2.5, 3, 4, or 5, or a value between the highest and lowest of the series |
| | **Total weight of Blend** | **100** |

| **S. No.** | **Ingredients** | **SIL/F1** | | **Alternative formulations** |
|---|---|---|---|---|
| | **Stage** - **A (Blending)** | **mg/capsule** | **wt%** | **wt%** |
| 1. | Silodosin | 4.00 | 2.3 | 2-3 |
| 2. | Mannitol (Pearlitol SD 200) | 132.40 | 75.7 | 70-80 |
| 3. | Low-substituted Hydroxypropylcellulose (L-HPC LH-11) | 35.00 | 20 | 15-25 |
| 4. | Sodium Lauryl Sulphate | 1.80 | 1 | 0.5-2 |
| 5. | Magnesium Stearate | 1.80 | 1 | 0.5-2 |
| | **Total weight of Blend** | **175.00** | **100** | **100** |

| **S. No.** | **Ingredients** | **SIL/F2** | | **Alternative formulations** |
|---|---|---|---|---|
| | **Stage** - **A (Blending)** | **mg/capsule** | **wt%** | **wt%** |
| 1. | Silodosin | 4.00 | 2.7 | 2-3 |
| 2. | Mannitol (Pearlitol SD 200) | 97.40 | 64.9 | 60-70 |
| 3. | Low-substituted Hydroxypropylcellulose (L-HPC LH-21) | 45.00 | 30 | 25-35 |
| 4. | Sodium Lauryl Sulphate | 1.80 | 1.2 | 0.5-2 |
| 5. | Magnesium Stearate | 1.80 | 1.2 | 0.5-2 |
| | **Total weight of Blend** | **150.00** | **100** | **100** |

| **S. No.** | **Ingredients** | **SIL/F3** | | **Alternative formulations** |
|---|---|---|---|---|
| | **Stage** - **A (Blending)** | **mg/capsule** | **wt%** | **wt%** |
| 1. | Silodosin | 4.00 | 2.3 | 2-3 |
| 2. | Mannitol (Pearlitol SD 200) | 87.40 | 50 | 40-60 |
| 3. | Low-substituted Hydroxypropylcellulose (L-HPC LH-21) | 80.00 | 45.7 | 40-50 |
| 4. | Sodium Lauryl Sulphate | 1.80 | 1 | 0.5-2 |
| 5. | Magnesium Stearate | 1.80 | 1 | 0.5-2 |
| | **Total weight of Blend** | **175.00** | **100** | **100** |

In one embodiment the diluent is sorbitol and the disintegrant is PGS, and wherein the ratio of diluent to disintegrant in the mixture is from 5:1 to 1:10.

In one embodiment of the invention, the pharmaceutical formulation is characterized in that
a) the silodosin is present in an amount of 1-4 wt%, preferably 2-4 wt%,
b) the surfactant is SLS and is present in an amount of 0.1-5 wt%, preferably 0.5-2 wt%,
c) the lubricant is magnesium stearate and is present in an amount of 0.1-5 wt%, preferably 0.5-2 wt%,
d) the disintegrant is L-HPC according to the claims and further PGS which is present in an amount 20-90 wt%, preferably 25-90 wt%, and
e) the diluent is sorbitol and is present in an amount of 5-80 wt%, based on the total weight of all components of the mixture.

The formulation described above relates preferably to the specific formulations SIL/F6, SIL/F7, SIL/F8. As demonstrated below, these formulations enable surprisingly beneficial API stability, dissolution and blend handling as described herein.

A formulation relates preferably to SIL/F6, SIL/F7, SIL/F8, or alternative formulations based closely on SIL/F6, SIL/F7, or SIL/F8, as demonstrated below. Only embodiments comprising L-HPC in an amount of 16%wt or more are according to the claims.

| | **Ingredients** | **wt%** |
|---|---|---|
| 1. | Silodosin | 1.0, 1.5, 2.0, 2.3, 2.5, 3.0, 3.5 or 4.0, or a value between the highest and lowest of the series |
| 2. | Diluent, preferably sorbitol | 5, 10, 15, 20, 25, 30, 35, 40, 45, 50, 55, 60, 65, 70, 75 or 80, or a value between the highest and lowest of the series |
| 3. | Disintegrant, preferably PGS (with L-HPC present according to the claims) | 16, 20, 25, 30, 35, 40, 45, 50, 55, 60, 65, 70, 75, 80, 85 or 90, or a value between the highest and lowest of the series |
| 4. | Surfactant, preferably Sodium Lauryl Sulphate | 0.1, 0.5, 0.75, 1.0, 1.25, 1.5, 2, 2.5, 3, 4, or 5, or a value between the highest and lowest of the series |
| 5. | Lubricant, preferably Magnesium Stearate | 0.1, 0.5, 0.75, 1.0, 1.25, 1.5, 2, 2.5, 3, 4, or 5, or a value between the highest and lowest of the series |
| | **Total weight of Blend** | **100** |

| **S. No.** | **Ingredients** | **SIL/F6** | | **Alternative formulations** |
|---|---|---|---|---|
| | **Stage - A (Blending)** | **mg/capsule** | **wt%** | **wt%** |
| 1. | Silodosin | 4.00 | 2.3 | 2-3 |
| 2. | Sorbitol (Neosorb P 100 T) | 17.50 | 10 | 5-15 |
| 3. | Pregelatinised starch (Starch PCS PC-10) | 43.00 | 24.6 | 20-30 |
| 4. | Pregelatinised starch (Starch 1500) | 106.90 | 61.1 | 50-70 |
| 5. | Sodium Lauryl Sulphate | 1.80 | 1 | 0.5-2 |
| 6. | Magnesium Stearate | 1.80 | 1 | 0.5-2 |
| | **Total weight of Blend** | **175.00** | **100** | **100** |

| **S. No.** | **Ingredients** | **SIL/F7** | | **Alternative formulations** |
|---|---|---|---|---|
| | **Stage** - **A (Blending)** | **mg/capsule** | **wt%** | **wt%** |
| 1. | Silodosin | 4.00 | 2.3 | 2-3 |
| 2. | Sorbitol (Neosorb P 100 T) | 132.40 | 75.6 | 70-80 |
| 3. | Pregelatinised starch (Starch PCS PC-10) | 26.00 | 15 | 10-20 |
| 4. | Pregelatinised starch (Starch 1500) | 9.00 | 5.1 | 2-8 |
| 5. | Sodium Lauryl Sulphate | 1.80 | 1 | 0.5-2 |
| 6. | Magnesium Stearate | 1.80 | 1 | 0.5-2 |
| | **Total weight of Blend** | **175.00** | **100** | **100** |

| **S. No.** | **Ingredients** | **SIL/F8** | | **Alternative formulations** |
|---|---|---|---|---|
| | **Stage - A (Blending)** | **mg/capsule** | **wt%** | **wt%** |
| 1. | Silodosin | 4.00 | 2.3 | 2-3 |
| 2. | Sorbitol (Neosorb P 100 T) | 87.40 | 50 | 45-55 |
| 3. | Pregelatinised starch (Starch PCS PC-10) | 71.00 | 40.6 | 35-45 |
| 4. | Pregelatinised starch (Starch 1500) | 9.00 | 5.1 | 2-8 |
| 5. | Sodium Lauryl Sulphate | 1.80 | 1 | 0.5-2 |
| 6. | Magnesium Stearate | 1.80 | 1 | 0.5-2 |
| | **Total weight of Blend** | **175.00** | **100** | **100** |

Furthermore disclosed is a pharmaceutical formulation that is characterized in that
a) the silodosin is present in an amount of 1-4 wt%, preferably 2-4 wt%,
b) the surfactant is SLS and is present in an amount of 0.1-5 wt%, preferably 0.5-2 wt%,
c) the lubricant is magnesium stearate and is present in an amount of 0.1-5 wt%, preferably 0.5-2 wt%,
d) the disintegrant is MCC and is present in an amount 30-70 wt%, and
e) the diluent is mannitol and is present in an amount of 30-70 wt%, based on the total weight of all components of the mixture.

In one embodiment of the invention, the pharmaceutical formulation according to the claims is characterized in that
a) the silodosin is present in an amount of 1-4 wt%, preferably 2-4 wt%,
b) the surfactant is SLS and is present in an amount of 0.1-5 wt%, preferably 0.5-2 wt%,
c) the lubricant is magnesium stearate and is present in an amount of 0.1-5 wt%, preferably 0.5-2 wt%,
d) the disintegrant is L-HPC and is present in an amount 16-30 wt%, and
e) the diluent is sorbitol and is present in an amount of 60-90% wt%, based on the total weight of all components of the mixture.

The formulations described above relate preferably to the specific formulations SIL/F4, and SIL/F5. As demonstrated below, these formulations enable surprisingly beneficial API stability, dissolution and blend handling as described herein.

In further embodiments of the invention the formulation relates preferably to SIL/F4, or SIL/F5, or alternative formulations based closely on SIL/F4, or as demonstrated below. Only embodiments comprising L-HPC in an amount of 16%wt or more are according to the claims.

| | **Ingredients** | **wt%** |
|---|---|---|
| 1. | Silodosin | 1.0, 1.5, 2.0, 2.3, 2.5, 3.0, 3.5 or 4.0, or a value between the highest and lowest of the series |
| 2. | Diluent, preferably sorbitol or mannitol | 30, 35, 40, 45, 50, 55, 60, 65, 70, 75 or 80, or a value between the highest and lowest of the series |
| 3. | Disintegrant, preferably MCC or L-HPC | 16, 20, 25, 30, 35, 40, 45, 50, 55, 60 or 65, or a value between the highest and lowest of the series |
| 4. | Surfactant, preferably Sodium Lauryl Sulphate | 0.1, 0.5, 0.75, 1.0, 1.25, 1.5, 2, 2.5, 3, 4, or 5, or a value between the highest and lowest of the series |
| 5. | Lubricant, preferably Magnesium Stearate | 0.1, 0.5, 0.75, 1.0, 1.25, 1.5, 2, 2.5, 3, 4, or 5, or a value between the highest and lowest of the series |
| | **Total weight of Blend** | **100** |

| **S. No.** | **Ingredients** | **SIL/F4** | | **Alternative formulations** |
|---|---|---|---|---|
| | **Stage** - **A (Blending)** | **mg/capsule** | **wt%** | **wt%** |
| 1. | Silodosin | 4.00 | 2.3 | 2-3 |
| 2. | Mannitol (Pearlitol SD 200) | 84.15 | 48.1 | 40-60 |
| 3. | Cellulose, microcrystalline (Comprecel M 102D+) | 84.15 | 48.1 | 40-60 |
| 4. | Sodium Lauryl Sulphate | 1.80 | 1 | 0.5-2 |
| 5. | Magnesium Stearate | 0.90 | 0.5 | 0.5-2 |
| | **Total weight of Blend** | **175.00** | **100** | **100** |

| **S. No.** | **Ingredients** | **SIL/F5** | | **Alternative formulations** |
|---|---|---|---|---|
| | **Stage** - **A (Blending)** | **mg/capsule** | **wt%** | **wt%** |
| 1. | Silodosin | 4.00 | 2.3 | 2-3 |
| 2. | Sorbitol (Neosorb P 100 T) | 132.40 | 75.7 | 70-80 |
| 3. | Low-substituted Hydroxypropylcellulose (L-HPC LH-11 ) | 35.00 | 20 | 15-25 |
| 4. | Sodium Lauryl Sulphate | 1.80 | 1 | 0.5-2 |
| 5. | Magnesium Stearate | 1.80 | 1 | 0.5-2 |
| | **Total weight of Blend** | **175.00** | **100** | **100** |

Furthermore disclosed is a pharmaceutical formulation that is characterized in that
a) the silodosin is present in an amount of 1-4 wt%, preferably 2-4 wt%,
b) the surfactant is SLS and is present in an amount of 0.1-5 wt%, preferably 0.5-2 wt%,
c) the lubricant is magnesium stearate and is present in an amount of 0.1-5 wt%, preferably 0.5-2 wt%, and
d) the disintegrant is PGS and is present in an amount 80-97 wt%, based on the total weight of all components of the mixture.

The formulations described above relate preferably to the specific formulation SIL/F9. As demonstrated below, this formulation enables surprisingly beneficial API stability, dissolution and blend handling as described herein.

In further embodiments of the invention the formulation relates preferably to SIL/F9, or alternative formulations based closely on SIL/F9, as demonstrated below. Only embodiments comprising L-HPC in an amount of 16%wt or more are according to the claims.

| **S. No.** | **Ingredients** | **SIL/F9** | | **Alternative formulations** |
|---|---|---|---|---|
| | **Stage** - **A (Blending)** | **mg/capsule** | **wt%** | **wt%** |
| 1. | Silodosin | 4.00 | 2.3 | 2-3 |
| 2. | Pregelatinised starch (Starch PCS PC-10) | 83.40 | 47.7 | 40-50 |
| 3. | Pregelatinised starch (Starch 1500) | 84.00 | 48 | 40-50 |
| 4. | Sodium Lauryl Sulphate | 1.80 | 1 | 0.5-2 |
| 5. | Magnesium Stearate | 1.80 | 1 | 0.5-2 |
| | **Total weight of Blend** | **175.00** | **100** | **100** |

In one embodiment of the invention the pharmaceutical composition is characterized in that the composition does not comprise an alkaline inorganic salt and/or basic co-polymer.

In one embodiment of the invention the pharmaceutical composition is characterized in that the composition does not comprise sodium stearyl fumarate and/or cyclodextrin.

The prior art teaches that increased stability is often only possible via the application of additional stabilizers such as alkaline metal salts, alkaline inorganic salt or basic co-polymers as basic stabilizers. The beneficial stability of silodosin in the formulations described herein, that do not employ additional stabilizers, such as those described in WO 2015/152680, WO 2012/010669 or WO 2012/000926, could not have been predicted due to any pointer in the prior art.

In one embodiment of the invention the pharmaceutical composition does not comprise an alkaline inorganic salt employed as a stabilizer, such as those disclosed in WO 2015/152680, such as oxides, hydroxides, carbonates and phosphates of sodium, calcium, potassium, magnesium, manganese, iron, copper, zinc, aluminum and lithium. In one embodiment, the alkaline inorganic salt not included in the composition can be selected from the group consisting of alkaline metal or alkaline earth metal oxide, hydroxide, carbonate, and phosphate. In another embodiment, the alkaline inorganic salt not included in the composition can be calcium carbonate (CaCO₃), magnesium carbonate (MgCO₃), sodium hydrogen carbonate (NaHCO₃), or a mixture thereof.

In one embodiment of the invention the pharmaceutical composition does not comprise one or more basic copolymers, such as those disclosed in WO 2012/010669, for example having monomer units derived from methyl methacrylate, butyl methacrylate and dimethylaminoethyl methacrylate. Such basic copolymers are, for example, commercially available under the trade name Eudragit® E, in particular the products Eudragit® E 100, Eudragit® E, Eudragit® E 12,5.

In contrast to the teachings of the prior art, the present invention is not dependent on employing basic components as stabilizers. The formulations of the present invention are stable despite the main excipients, i.e. diluents like mannitol or sorbitol and disintegrants like PGS and L-HPC, being weak acids, typically with pH 5-7 according to Handbook of Pharmaceutical Excipients. Surprisingly, basic compounds are not required in order to main stability of silodosin over long periods of time, as is evidenced by the 6 month stability data presented below.

In one embodiment of the invention the pharmaceutical composition does not comprise one or more cyclodextrins, such as those disclosed in WO 2012/000926, wherein cyclodextrins are a class of compounds belonging to the cyclic oligosaccharides. The ring of six glucose units is also called α-cyclodextrin, the ring of seven glucose units is called β-cyctodextrin, and the ring of eight glucose units is called γ-cyclodextrin. In one embodiment, the cyclodextrin not included in the composition can be selected from α-, β- and γ-cyclodextrin.

In one embodiment of the invention, the capsule is a light shielding capsule, preferably comprising titanium dioxide. Silodosin is known as a light-sensitive compound. The invention therefore employs preferably in some embodiments a light shielding capsule in order to further reduce any instability of the API.

A further aspect of the invention relates to the therapeutic use of the pharmaceutical composition as described herein in the treatment of dysuria and/or benign prostatic hyperplasia. The invention therefore relates to a method for the treatment of dysuria, benign prostatic hyperplasia and/or symptoms thereof, comprising the administration of a therapeutically effective amount to a subject in need thereof.

A further aspect of the invention relates to a method of preparing a pharmaceutical composition in the form of a capsule, said capsule comprising a powder mixture of silodosin, a diluent, a disintegrant, a surfactant and a lubricant, wherein said methods comprises dry mixing or blending the silodosin, diluent, disintegrant, surfactant and lubricant in a single powder mixture or blend, optionally sifting the mixture or blend, followed by direct filling into a capsule, wherein the method preferably does not comprise granulation.

In one embodiment the method comprises mixing and sifting of the API with the pharmaceutical excipients disclosed herein in a single mixture. The mixture is subsequently blended in order to produce a homogenous powder blend or mixture, which is subsequently filled directly into appropriate capsules, preferably hard gelatin capsules. Post-filling the capsules are sealed and packaged, preferably using PVC/PVdC blister packaging.

As described above, the method of the present invention is unexpectedly advantageous due to its simplicity and is enabled by the surprising beneficial properties of the formulations described herein. It was unknown and unexpected for a skilled person that a straightforward method of manufacture comprising direct filling of a powder blend comprising silodosin into a capsule could be employed that would lead to formulations with beneficial stability of silodosin, sufficient dissolution and good content uniformity.

In further embodiments of the invention granulation is not employed in the method of manufacture. In contrast to the teachings of the prior art, which suggest primarily wet granulation processes, it was found by the inventors that granulation processes could be avoided entirely when producing the compositions described herein.

### DETAILED DESCRIPTION OF THE INVENTION

Silodosin (1-(3-hydroxypropyl)-5-[(2R)-({2-[2-[2-(2,2,2-trifluoroethoxy)phenoxy]ethyl}amino) propyl]indoline-7-carboxamide) has a known selective suppression effect of the urethra smooth muscle contraction and is an extremely useful compound as the medicament for dysuria treatment. Silodosin is highly selective for α1A-adrenoreceptors that are primarily located in the human prostate, bladder base, bladder neck, prostatic capsule and prostatic urethra. Blockade of these α1A-adrenoreceptors causes smooth muscle in these tissues to relax, thus decreasing bladder outlet resistance, without affecting detrusor smooth muscle contractility. This causes an improvement of both storage (irritative) and voiding (obstructive) symptoms (lower urinary tract symptoms, LUTS) associated with benign prostatic hyperplasia.

Daily standard dose of silodosin for adults is 1 to 16 mg, which can be administered in an amount of 1 mg, 2 mg, 4 mg, 8 mg, or 16 mg per day. The compositions of the present invention preferably employ 4 mg or 8 mg of silodosin in a single capsule.

The term "silodosin" as used herein according to the present invention includes silodosin in the form of free base, a pharmaceutically acceptable salt thereof, amorphous silodosin, crystalline silodosin, preferably selected from the polymorph forms described herein, any isomer, derivative, hydrate, solvate or prodrug or a combination thereof.

Silodosin may also be prepared as a pharmaceutical salt. Examples of pharmaceutical acceptable salts of silodosin which can be contained as an active ingredient in a solid oral dosage form include acid addition salt formed with mineral acids such as hydrochloric acid, hydrobromic acid, hydroiodic acid, sulfuric acid, nitric acid, phosphoric acid and the like; acid addition salts formed with organic acids such as acetic acid, propionic acid, butyric acid, oxalic acid, citric acid, succinic acid, tartaric acid, fumaric acid, malic acid, lactic acid, adipic acid, benzoic acid, salicylic acid, methanesulfonic acid, benzenesulfonic acid, p-toluenesulfonic acid, glutamic acid, aspartic acid and the like. Examples of solvate include solvates with water, ethyl alcohol or the like.

Various forms of silodosin may be employed, for example in amorphous form or in the crystal forms described in EP 1541 554.

Silodosin has at least three crystal forms shown by the powder X-ray diffraction patterns in EP 1541554, namely:
(1) a crystal characterized by main peaks of 5. 5 DEG +/- 0.2 DEG , 6.1 DEG +/- 0.2 DEG , 9.8 DEG +/- 0.2 DEG , 11.1 DEG +/- 0.2 DEG , 12. 2 DEG +/- 0.2 DEG , 16.4 DEG +/- 0.2 DEG , 19.7 DEG +/- 0.2 DEG and 20.0 DEG +/- 0.2 DEG as 2[theta] (commonly referred to as crystal form alpha);
(2) a crystal characterized by main peaks of 7.0 DEG +/- 0.2 DEG, 12.5 DEG +/- 0.2 DEG, 18.5 DEG +/- 0.2 DEG, 19.5 DEG +/- 0.2 DEG, 20.7 DEG +/- 0.2 DEG and 21.1 DEG +/- 0.2 DEG as 2[theta] (commonly referred to as crystal form beta); and
(3) a crystal characterized by main peaks of 6.0 DEG +/- 0.2 DEG, 10.6 DEG +/- 0.2 DEG, 12.6 DEG +/- 0.2 DEG, 17.1 DEG +/- 0.2 DEG, 17.9 DEG +/- 0.2 DEG, 20.7 DEG +/- 0.2 DEG and 23.7 DEG +/- 0.2 DEG as 2[theta] (commonly referred to as crystal form gamma).

Methods for the preparation of silodosin crystal forms are described in EP 1541554. In a preferred embodiment of the invention the crystal form beta is employed in the composition of the present invention. As demonstrated below, the compositions of the invention show unexpectedly good stability after 6 months storage with respect to maintaining the polymorph employed in the compositions. Good polymorph stability may be demonstrated for any polymorph form, especially for form alpha or beta, preferably for form beta.

The term "active ingredient" or "API" herein refers to a pharmaceutically active molecule (e.g. silodosin) as well as its pharmaceutically acceptable and therapeutically active salts, esters, amides, prodrugs, metabolites, enantiomers, polymorphs, analogs, etc. that induce a desired pharmacological or physiological effect. Terms like "active", "active agent", "active substance" may be used synonymously for "active ingredient".

The term "effective amount" or "therapeutically effective amount" used interchangeably, is defined to mean the amount or quantity of the active drug (e.g. silodosin), which is sufficient to elicit an appreciable biological response when administered to the patient. It will be appreciated that the precise therapeutic dose will depend on the age and condition of the patient, nature of the condition to be treated and will be at the ultimate discretion of the attendant physician.

The term "excipient" means a pharmacologically inactive component such as a diluent, disintegrant, carrier, and the like, of a pharmaceutical product. The excipients that are useful in preparing a pharmaceutical composition are generally safe, non-toxic and are acceptable for veterinary as well as human pharmaceutical use. Reference to an excipient includes both one excipient and more than one excipient.

The excipients are described herein in some embodiments according to "wt%", or "percentage by weight". The %wt values recite herein preferably relate to the percentage of material by weight present in the powder mixture, or powder blend. The weight percentages recited as %wt based on the total weight of all components of the mixture, is intended to convey the % of material in the powder, disregarding the weight of the capsule shell itself, such as the hard gelatin capsule into which the powder is filled.

According to the present invention, a disintegrant is typically an agent used in the preparation of solid pharmaceutical formulations which causes them to disintegrate and release their medicinal substances on contact with moisture. Disintegrants include but are not limited to hydroxypropyl cellulose (L-HPC), pregelatinized starch (PGS), crospovidone, microcrystalline cellulose (MCC), croscarmellose sodium, sodium starch glycolate and the like. Various disintegrants may also function as binders, such as pregelatinized starch, hydroxypropyl cellulose, and hydroxypropyl methylcellulose. According to the claims, L-HPC is present as a disintegrant in an amount of 16%wt or more.

According to the invention, a diluent (or filler) may be used as a bulking agent. Various useful fillers or diluents include but are not limited to mannitol, sorbitol, xylitol, and the like, and mixtures thereof; more preferably selected from mannitol and sorbitol.

According to the present invention, one or more lubricants, which improve the flow of a powder blend, can be used. Useful lubricants include but are not limited to, magnesium stearate, sodium stearyl fumarate, colloidal silicon dioxide, magnesium silicate, magnesium trisilicate, talc, and other forms of silicon dioxide, such as aggregated silicates and hydrated silica.

According to the present invention, surfactants (also termed as surface-active agents also wetting agents, emulsifying agents or suspending agents) are substances which adsorb onto the surfaces or interfaces of a system and lower the surface tension of the medium in which it is dissolved, and/or the interfacial tension with other phases. Suitable surfactants may include, but are not limited to, anionic, cationic, non-ionic or amphoteric surfactants, preferably sodium lauryl sulfate (SLS), or other surfactants known to the person skilled in the art.

The invention relates further to a method for treating dysuria and symptoms thereof by administration of the composition of the present invention. The scope of the invention is defined by the claims. Any references in the description to methods of treatment refer to the compounds, pharmaceutical compositions and medicaments of the present invention for use in a method for treatment of the human (or animal) body by therapy (or for diagnosis).

Dysuria is associated with a disorder of urination control nerves in the urethra or the bladder, for example encephalopathy. These disorders may occur in both men and women and are generally called as neurogenic bladder. Dysuria is also associated with urethra functional obstruction that does not accompany a disorder of urination control nerves. As such, dysuria may also be caused by urination difficulty, bladder cervix blockage, urethra syndrome, detrusor muscle-sphincter muscle cooperation insufficiency, chronic cystitis, prostatodynia, Hinman syndrome, Fowler syndrome, psychogenic dysuria, drug-induced dysuria, aging and the like, besides bladder cervix sclerosis, chronic prostatitis and unstable bladder. These disorders are generally termed lower urinary tract disorders. Accordingly, the medicaments of the present invention are extremely useful as agents for the treatment of dysuria associated with urethra organized obstruction, such as prostate hypertrophy, urethra stricture, urethra calculus and prostate cancer; dysuria caused by disorder of urination control nerves, namely neurogenic bladder; and dysuria caused by urethra functional obstruction, namely lower urinary tract disorders.

The invention further relates to a method for manufacture of the compositions described herein. In embodiments of the invention the method is carried out by direct filling of a capsule with the powder blend described herein.

According to the invention, a powder or powder blend is a substantially dry solid, composed of preferably a large number of fine particles that may flow when shaken or tilted. The powder of the present invention is preferably distinguishable from the silodosin granules of the prior art, which are typically prepared by wet or dry granulation, and are typically larger than the particles of the powder of the invention.

Suitable devices for machine filling of a powder are known to one skilled in the art. For optimum machine-filling performance, the powder must be of the right flow and density; the densities of the excipients and the drug should therefore be similar. As described herein, the combination of excipients of the formulations of the invention are suitable for use in combination in order to achieve suitable flow characteristics.

Capsule direct filing can be carried out, in some embodiments, by using an automated or high speed filling machines. Various devices suitable for direct filling are known to a skilled person, such as those disclosed in Stegemann & Bornem ("Hard gelatin capsules today - and tomorrow", 2nd edition, 2002). Such machines typically rely on two filling principles referred to as "dosator type" or "dosing disk or tamping type", either of which may be employed by the present invention. The dosator principle uses dosing tube that dip in a powder bed that is normally two times higher than the final plug length. During the dipping and by the dosator piston movement, the powder is densified to form a cohesive plug. The dosing tube transfers the plug to the capsule body for ejection. The dosing disk principle is based on filling chambers that are bored into the dosing disk. Powder flows into the filling chambers followed by a slight compression by a tamping punch, which is repeated five times before the plug is ejected into the capsule body through the hole of the tamping disk.

The intended composition of the present invention is a capsule, preferably a hard gelatin capsule. Capsules are typically defined as solid preparations with hard or soft shells of various shapes and capacities, usually containing a single dose of active ingredient, intended for oral administration.

In a preferred embodiment, hard capsules have shells consisting of two cylindrical sections, one end of which is rounded and closed, the other open. The active ingredient or ingredients, usually in solid form (powder) are filled into one of the sections which is then closed by slipping the other section over it. The security of the closure may be strengthened by suitable means. Capsules may also be described as solid dosage forms in which the active ingredients are sealed in a hard or soft container or shell. In light of automatic filling machines, capsules may be provided in various formats, for example in a single operation, automatic capsule filling machines line up and rectify the hard gelatin capsules, separate body and cap, fill the body, join cap and body together (for closing), and eject the filled capsule. Hard gelatin capsules are preferred, as they are simple in their formulation and production and their disintegration is both known and controllable. Hard gelatin capsules are easier and quicker to formulate and produce, whatever the batch size, compared with other solid oral dosage forms. Selection of a suitable capsule can be carried out by means known to a skilled person, for example using guidance obtained Stegemann & Bornem ("Hard gelatin capsules today - and tomorrow", 2nd edition, 2002).

Silodosin is known to be light-sensitive, thereby leading potentially to impurities. As a result, titanium oxide is most preferred as a light-shielding material, and highly photo stable solid dosage form pharmaceuticals are prepared by using capsules containing titanium oxide or coating agents containing titanium oxide. Light-shielding effects increase with blending amounts of titanium oxide while the strength of capsules decreases with blending amounts of titanium oxide. Preferred blending amounts are appropriately determined depending on the size of pharmaceuticals. For exerting preferable light-shielding effects in capsules, the blending amount of titanium oxide is not less than about 3%, more preferably about 3.4-3.6%. Alternatively, the blending amount of titanium oxide is determined by the surface area of the composition, the amount of coating agents and the like. For exerting preferable light-shielding effects, the coating amount of titanium oxide is generally not less than 0.5mg/square cm, more preferably 1.1mg/square cm based on the surface area of tablets.

### EXAMPLES

The invention is demonstrated by way of the examples disclosed herein. The examples provide technical support for and a more detailed description of potentially preferred, nonlimiting embodiments of the invention.

### Part A

### Comparative example 1 (SIL/C1):

SIL/C1 was produced by following essentially the method according to EP 1574215 in order to produce a capsule of wet-granulated silodosin granules, in which sodium lauryl sulfate and magnesium stearate were added post-granulation.

Silodosin, mannitol, and pregelatinized starch were sufficiently mixed, followed by the wet granulation process using purified water as granulation liquid. The granulated mixture was dried with an air dryer at 60°C until the LOD is not more than 1.5% w/w and the granules were sieved. Sodium lauryl sulfate and magnesium stearate were added and mixed for 5 min and filled into empty hard gelatin capsule shells (size 3).

| **S. No.** | **Ingredients** | **SIL/C1** |
|---|---|---|
| | | **mg/capsule** |
| **Stage-A: Intra granular** | | |
| 1 | Silodosin | 4.00 |
| 2 | D-Mannitol (Pearlitol 200 SD) | 132.40 |
| 3 | Starch, pregelatinised (Starch 1500) | 9.00 |
| 4 | Starch, pregelatinised (Starch PCS PC-10) | 26.00 |

| **Stage-B: Granulation** | | |
|---|---|---|
| 5 | Water, Purified | Q.S. |

| **Stage-C: Extra granular** | | |
|---|---|---|
| 6 | Sodium lauryl sulfate | 1.80 |
| 7 | Magnesium stearate | 1.80 |
| **Total weight of Blend** | | **175.00** |

### Comparative example 2 (Urorec 4 mg; SIL/C2)

Urorec® capsules were obtained for analysis of silodosin stability, as described in more detail below. The impurity profile of freshly procured and stored reference product Urorec® 4 mg (lot SE4L53, exp. 02-2018) is presented in the table below:

**Table: Summary of impurity profiles at 40°C/75%RH**

| **Batch No.** | **Stage/ Condition** | **Pack** | **Impurity (%m/m)** | | |
|---|---|---|---|---|---|
| | | | **Impurity-A** | **Max. Unknown** | **Total impurities** |
| **SIL/C2** | Initial 6 Months | White opaque PVC/ PVdC - blister | 0.14 1.51 | 0.02 0.14 | 0.20 1.72 |

### Part B

### General procedure for the preparation of inventive examples below employing a dry mixing process

Silodosin and all further excipients were sifted, sufficiently mixed in a blender for about 15 min and filled into empty hard gelatin capsule shells.

**Brief Manufacturing Process:**

| | |
|---|---|
| 1.0 | Dispense the materials according to the indicated amounts |
| 2.0 | Sift Silodosin along with Mannitol, Hydroxypropylcellulose, low-substituted, Sodium laurilsulfate and Magnesium stearate, through a #30 mesh. |
| 3.0 | Load the material of 2.0 in a blender and mix for sufficient time. |
| 4.0 | Fill the blend into empty hard gelatin capsules using suitable machine parameters. |

The following compositions were obtained using the above process:

**Example 1 (SIL/F1):**

| **S. No.** | **Ingredients** | **SIL/F1** | |
|---|---|---|---|
| | **Stage** - **A (Blending)** | **mg/capsule** | **wt%** |
| 1. | Silodosin | 4.00 | 2.3 |
| 2. | Mannitol (Pearlitol SD 200) | 132.40 | 75.7 |
| 3. | Low-substituted Hydroxypropylcellulose (L-HPC LH-11 ) | 35.00 | 20 |
| 4. | Sodium Lauryl Sulphate | 1.80 | 1 |
| 5. | Magnesium Stearate | 1.80 | 1 |
| | **Total weight of Blend** | **175.00** | **100** |

| | **Stage-B: (Encapsulation)** | **Capsule size:** | |
|---|---|---|---|
| 1. | Empty Hard Gelatin Capsule Shells | Size 3 | |

**Example 2 (SIL/F2):**

| **S. No.** | **Ingredients** | **SIL/F2** | |
|---|---|---|---|
| | **Stage** - **A (Blending)** | **mg/capsule** | **wt%** |
| 1. | Silodosin | 4.00 | 2.7 |
| 2. | Mannitol (Pearlitol SD 200) | 97.40 | 64.9 |
| 3. | Low-substituted Hydroxypropylcellulose (L-HPC LH-21 ) | 45.00 | 30 |
| 4. | Sodium Lauryl Sulphate | 1.80 | 1.2 |
| 5. | Magnesium Stearate | 1.80 | 1.2 |
| | **Total weight of Blend** | **150.00** | **100** |

| | **Stage-B: (Encapsulation)** | **Capsule size:** | |
|---|---|---|---|
| 1. | Empty Hard Gelatin Capsule Shells | Size 3 | |

The same blend (300 mg) is alternatively filled in empty hard gelatin capsule shells (size 1) to produce 8 mg silodosin capsules.

**Example 3 (SIL/F3):**

| **S. No.** | **Ingredients** | **SIL/F3** | |
|---|---|---|---|
| | **Stage - A (Blending)** | **mg/capsule** | **wt%** |
| 1. | Silodosin | 4.00 | 2.3 |
| 2. | Mannitol (Pearlitol SD 200) | 87.40 | 50 |
| 3. | Low-substituted Hydroxypropylcellulose (L-HPC LH-21) | 80.00 | 45.7 |
| 4. | Sodium Lauryl Sulphate | 1.80 | 1 |
| 5. | Magnesium Stearate | 1.80 | 1 |
| | **Total weight of Blend** | **175.00** | **100** |

| | **Stage-B: (Encapsulation)** | **Capsule size:** | |
|---|---|---|---|
| 1. | Empty Hard Gelatin Capsule Shells | Size 3 | |

**Comparative Example 4 (SIL/F4):**

| **S. No.** | **Ingredients** | **SIL/F4** | |
|---|---|---|---|
| | **Stage** - **A (Blending)** | **mg/capsule** | **wt%** |
| 1. | Silodosin | 4.00 | 2.3 |
| 2. | Mannitol (Pearlitol SD 200) | 84.15 | 48.1 |
| 3. | Cellulose, microcrystalline (Comprecel M 102D+) | 84.15 | 48.1 |
| 4. | Sodium Lauryl Sulphate | 1.80 | 1 |
| 5. | Magnesium Stearate | 0.90 | 0.5 |
| | **Total weight of Blend** | **175.00** | **100** |

| | **Stage-B: (Encapsulation)** | **Capsule size:** | |
|---|---|---|---|
| 1. | Empty Hard Gelatin Capsule Shells | Size 2 | |

**Example 5 (SIL/F5):**

| **S. No.** | **Ingredients** | **SIL/F5** | |
|---|---|---|---|
| | **Stage** - **A (Blending)** | **mg/capsule** | **wt%** |
| 1. | Silodosin | 4.00 | 2.3 |
| 2. | Sorbitol (Neosorb P 100 T) | 132.40 | 75.7 |
| 3. | Low-substituted Hydroxypropylcellulose (L-HPC LH-11) | 35.00 | 20 |
| 4. | Sodium Lauryl Sulphate | 1.80 | 1 |
| 5. | Magnesium Stearate | 1.80 | 1 |
| | **Total weight of Blend** | **175.00** | **100** |

| | **Stage-B: (Encapsulation)** | **Capsule size:** | |
|---|---|---|---|
| 1. | Empty Hard Gelatin Capsule Shells | Size 3 | |

**Comparative Example 6 (SIL/F6):**

| **S. No.** | **Ingredients** | **SIL/F6** | |
|---|---|---|---|
| | **Stage** - **A (Blending)** | **mg/capsule** | **wt%** |
| 1. | Silodosin | 4.00 | 2.3 |
| 2. | Sorbitol (Neosorb P 100 T) | 17.50 | 10 |
| 3. | Pregelatinised starch (Starch PCS PC-10) | 43.00 | 24.6 |
| 4. | Pregelatinised starch (Starch 1500) | 106.90 | 61.1 |
| 5. | Sodium Lauryl Sulphate | 1.80 | 1 |
| 6. | Magnesium Stearate | 1.80 | 1 |
| | **Total weight of Blend** | **175.00** | **100** |

| | **Stage-B: (Encapsulation)** | **Capsule size:** | |
|---|---|---|---|
| 1. | Empty Hard Gelatin Capsule Shells | Size 2 | |

**Comparative Example 7 (SIL/F7):**

| **S. No.** | **Ingredients** | **SIL/F7** | |
|---|---|---|---|
| | **Stage** - **A (Blending)** | **mg/capsule** | **wt%** |
| 1. | Silodosin | 4.00 | 2.3 |
| 2. | Sorbitol (Neosorb P 100 T) | 132.40 | 75.6 |
| 3. | Pregelatinised starch (Starch PCS PC-10) | 26.00 | 15 |
| 4. | Pregelatinised starch (Starch 1500) | 9.00 | 5.1 |
| 5. | Sodium Lauryl Sulphate | 1.80 | 1 |
| 6. | Magnesium Stearate | 1.80 | 1 |
| | **Total weight of Blend** | **175.00** | **100** |

| | **Stage-B: (Encapsulation)** | **Capsule size:** | |
|---|---|---|---|
| 1. | Empty Hard Gelatin Capsule Shells | Size 2 | |

**Comparative Example 8 (SIL/F8):**

| **S. No.** | **Ingredients** | **SIL/F8** | |
|---|---|---|---|
| | **Stage** - **A (Blending)** | **mg/capsule** | **wt%** |
| 1. | Silodosin | 4.00 | 2.3 |
| 2. | Sorbitol (Neosorb P 100 T) | 87.40 | 50 |
| 3. | Pregelatinised starch (Starch PCS PC-10) | 71.00 | 40.6 |
| 4. | Pregelatinised starch (Starch 1500) | 9.00 | 5.1 |
| 5. | Sodium Lauryl Sulphate | 1.80 | 1 |
| 6. | Magnesium Stearate | 1.80 | 1 |
| | **Total weight of Blend** | **175.00** | **100** |

| | **Stage-B: (Encapsulation)** | **Capsule size:** | |
|---|---|---|---|
| 1. | Empty Hard Gelatin Capsule Shells | Size 2 | |

**Comparative Example 9 (SIL/F9):**

| **S. No.** | **Ingredients** | **SIUF9** | |
|---|---|---|---|
| | **Stage** - **A (Blending)** | **mg/capsule** | **wt%** |
| 1. | Silodosin | 4.00 | 2.3 |
| 2. | Pregelatinised starch (Starch PCS PC-10) | 83.40 | 47.7 |
| 3. | Pregelatinised starch (Starch 1500) | 84.00 | 48 |
| 4. | Sodium Lauryl Sulphate | 1.80 | 1 |
| 5. | Magnesium Stearate | 1.80 | 1 |
| | **Total weight of Blend** | **175.00** | **100** |

| | **Stage-B: (Encapsulation)** | **Capsule size:** | |
|---|---|---|---|
| 1. | Empty Hard Gelatin Capsule Shells | Size 2 | |

### Part C

### Dissolution Study:

For the capsule formulations of inventive examples 1-9 and Comparative Examples 1-2, the dissolution test was carried out according to the following dissolution test method and conditions.

### Dissolution conditions and method:

The test was carried out using capsules placed into a sinker at a paddle speed of 50 revolutions per minute according to method 2 of dissolution test of the US Pharmacopeia (USP), using 900 mL of hydrochloric acid medium at pH 1.2. The temperature of the medium is maintained at 37°C ± 0.5°C using a water bath. Sample solutions were obtained at 5, 10, 15, 20, 30 and 45 minutes after starting the test, and filtered through a 0.45µm PVDF syringe filter.

### Dissolution analytical test method details:

**Equipment:** A High Performance Liquid Chromatographic system with gradient/isocratic elution capability, a spectrophotometric UV detector and an auto sampler was employed (Waters Alliance 2695 separations module, Waters 2487 dual λ absorbance detector or equivalent).

### Preparation of solutions:

**Standard stock solution:** A solution containing 0.22 mg/mL of Silodosin was prepared in diluent. 44.0 mg of Silodosin was weighed into a 200 mL clean, dry volumetric flask. 120 mL of diluent was added and sonicated to dissolve. The solution was made up to volume with diluent and mixed.

**Standard solution:** For 4 mg strength: A solution containing 0.044 mg/mL of Silodosin was prepared in dissolution medium. 2.0 mL of standard stock solution was diluted to 200 mL with dissolution medium and mixed. The solution was filtered through 0.45 µ PVDF syringe filter by discarding first 10mL of filtrate.

### HPLC chromatographic conditions:

Column: ACE 5 C18 PFP 250x4.6 mm, 5 µm or equivalent
Flow rate: 1.0 mL/min
Detection: UV, 270 nm
Injection Volume: 25 µL
Data acquisition time: 5 minutes
Pump mode: Isocratic
Column temperature: 30° C

**Results of dissolution tests:** All the formulations of inventive examples 1-9 and Comparative Examples 1-2 showed not less than 85% dissolution release after 15 minutes, as shown in the below table. Dissolution of the formulations of the present invention are comparable to the commercially available reference product (Urorec, SE4L53).

**Table: Results of dissolution tests:**

| ***Example*** | **% Release silodosin after 15 minutes** |
|---|---|
| **SIL/C2** | 98 |
| **SIL/F1** | 90 |
| **SIL/F5** | 100 |
| **SIL/F6** | 96 |
| **SIL/F7** | 100 |
| **SIL/F8** | 97 |

### Part D

### Stability Studies:

### Part D-1a: Chemical Stability/Impurity profiles:

A number of formulations were tested under accelerated conditions after open exposure for 2 days at 80°C/40%RH, and additionally in blisters after 6 months at 40°C/75% RH, for a defined time to test the presence of silodosin degradation products.

Degradation products were tested prior to storage and after storage under conditions as shown in tables for the corresponding examples. Degradation products were detected by HPLC analysis according to the following HPLC conditions.

### HPLC chromatographic conditions:

Column: Inert sustain C18 250 x 4.6 mm, 5µ particle size or equivalent
Flow rate: 1.15 mL/min
Detection: UV, 225 nm
Injection Volume: 20 µL
Data acquisition time: 60 minutes
Pump mode: Gradient
Column temperature: 40° C
Sample cooler temperature: 15° C

### Preparation of solutions:

**Standard solution:** A solution was prepared at a concentration of 0.00096 mg/mL of Silodosin. 24.0 mg of Silodosin was accurately weighted in a working standard into a 100 mL clean, dry volumetric flask, 60 mL of diluent was added and sonicated to dissolve. The solution was made up to volume with diluent and mixed. 5 mL of this solution was diluted to 50 mL with diluent and mixed. 4 mL of this solution was diluted to 100 mL with diluent and mixed. The solution was filtered through 0.45 µ PVDF syringe filter.

**Sample solution:** 10 capsules were opened and the contents of the capsules collected in a mortar and ground to a fine powder. The powder equivalent to 12 mg of Silodosin was weighed and transferred into a 25 mL clean and dry volumetric flask, 15 mL of diluent was added and sonicated for 10 minutes with intermittent shaking. The solution was made up to volume with diluent and mixed. The solution was filtered through 0.45 µ PVDF syringe filter by discarding first 10mL of filtrate.

**Placebo solution:** The powder equivalent to 525 mg of placebo was weighed and transferred into a 25 mL clean and dry volumetric flask, 15 mL of diluent was added and sonicated for 10 minutes with intermittent shaking. The solution was made up to volume with diluent and mixed. The solution was filtered through 0.45 µ PVDF syringe filter by discarding first 10mL of filtrate.

Impurity-A of Silodosin, also known as Dehydro impurity or (R)-1-(3-Hydroxypropyl)-5-(2-(2-(2-(2,2,2-trifluoroethoxy)phenoxy)ethylamino)propyl)-1H-indole-7-carboxamide (CAS number 175870-21-0), has the following chemical structure:

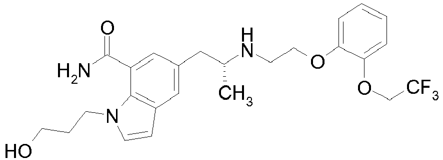

**Table: Accelerated stability data of capsules (open exposure):**

| **Example** | **Impurity (%m/m) initial** | | | **Impurity (%m/m) after 2 days at 80°C/40%RH** | | |
|---|---|---|---|---|---|---|
| | **Impurity-A** | **Max. Unknown** | **Total impurities** | **Impurity-A** | **Max. Unknown** | **Total impurities** |
| **SIL/C1** | 0.08 | 0.07 | 0.33 | 2.16 | 0.36 | 2.79 |
| **SIL/F1** | 0.02 | 0.07 | 0.20 | 1.10 | 0.12 | 1.51 |
| **SIL/F2** | 0.07 | BDL | 0.07 | 1.46 | 0.28 | 1.83 |
| **SIL/F5** | 0.02 | 0.07 | 0.20 | 0.92 | 0.12 | 1.12 |
| **SIL/F6** | 0.06 | 0.07 | 0.18 | 1.08 | 0.18 | 1.53 |
| **SIL/F7** | BDL | BDL | BDL | 0.92 | 0.18 | 1.33 |
| **SIL/F8** | BDL | BDL | BDL | 0.47 | 0.08 | 0.55 |

| | | | | | | |
|---|---|---|---|---|---|---|
| *BDL = below detection limit* | | | | | | |

**Table: Accelerated stability data of capsules in white opaque PVC/ PVdC blisters:**

| **Example** | **Impurity (%m/m) initial** | | | **Impurity (%m/m) after 6 months at 40°C/75%RH** | | |
|---|---|---|---|---|---|---|
| | **Impurity-A** | **Max. Unknown** | **Total impurities** | **Impurity-A** | **Max. Unknown** | **Total impurities** |
| **SIL/C2** | 0.14 | 0.02 | 0.20 | 1.51 | 0.14 | 1.72 |
| **SIL/F1** | 0.02 | 0.07 | 0.20 | 0.92 | 0.09 | 1.10 |
| **SIL/F5** | 0.02 | 0.07 | 0.20 | 1.26 | 0.25 | 1.70 |

As can be observed from the tables above, the novel formulations of the present invention show improved stability after 6 months compared to the commercial reference product (Urorec, as described above under batch number SE4L53). Urorec products demonstrated impurities (Impurity-A) of 1.51 % at 6 months, whereby the formulations of the present invention show levels of Impurity-A of 0.92% and 1.26%.

### Part D-1b: Chemical Stability/Impurity profiles of commercial formulations and comparative examples

A number of commercial formulations were tested under accelerated conditions (40°C/75%RH) in opaque PVC/PVdC blisters, in PVC/PE/PVdC blisters or in Alu-Alu blisters for a defined time to test the presence of silodosin degradation products. A number of commercial formulations were also tested under long-term conditions (25°C/60%RH) in opaque PVC/PVdC blisters, in PVC/PE/PVdC blisters or in Alu-Alu blisters for a defined time to test the presence of silodosin degradation products.

The silodosin degradation products were assessed as described above using HPLC. The results are demonstrated in the below tables.

**Table: Development trials with Mannitol & L-HPC (SIL/F1, SIL/F2) for use in stability studies.**

| **S.No.** | **Ingredients** | **SIL/F1** | **SIL/F1b alternative L-HPC grade** | **SIL/F2, commercial-001** |
|---|---|---|---|---|
| | **Blending and capsule filling** | **mg/caps ule** | **mg/capsule** | **mg/capsule** |
| 1 | Silodosin (Form β) | 4.00 | 4.00 | 4.00 |
| 2 | Mannitol (Pearlitol 200 SD) | 132.40 | 132.40 | 97.40 |
| 3 | Low-su bstituted Hydroxypropylcellulose (L-HPC LH-11) | 35.00 | - | - |
| 3 | Low-su bstituted Hydroxypropylcellulose (L-HPC LH-21) | - | 35.00 | 45.00 |
| 4 | Sodium Laurilsulfate | 1.80 | 1.80 | 1.80 |
| 5 | Magnesium stearate | 1.80 | 1.80 | 1.80 |
| | **Weight of blend** | **175.00** | **175.00** | **150.00** |

**Table: Development trial with Sorbitol & L-HPC (SIL/F5) for use in stability studies.**

| **S.No.** | **Ingredients** | **SIL/F5** |
|---|---|---|
| | **Blending and capsule filling** | **mg/tablet** |
| 1 | Silodosin (Form β) | 4.00 |
| 2 | Sorbitol (Neosorb P 100 T) | 132.40 |
| 3 | Low-substituted Hydroxypropylcellulose (L-HPC LH-11) | 35.00 |
| 4 | Sodium Laurilsulfate | 1.80 |
| 5 | Magnesium stearate | 1.80 |
| | **Weight of blend** | **175.00** |

**Table: Summary of impurity profiles at 40°C/75%RH**

| **Batch No.** | **Stage/Condition** | **Pack** | **Impurity (%m/m)** | | |
|---|---|---|---|---|---|
| | | | **Impurity-A** | **Max. Unknown** | **Total impurities** |
| **SIL/F1** | Initial | White opaque PVC/PVdC - blister | 0.02 | 0.07 | 0.20 |
| | 3 Months | | 0.28 | 0.06 | 0.45 |
| | 6 Months | | 0.92 | 0.09 | 1.10 |
| | Initial | Alu - Alu blister | 0.02 | 0.07 | 0.20 |
| | 3 Months | | 0.20 | 0.05 | 0.35 |
| | 6 Months | | 0.59 | 0.06 | 0.65 |
| **SIL/F2, comm001** | Initial | White opaque PVC/PVdC - blister | 0.04 | BDL | 0.04 |
| | 3 Months | | 0.27 | BDL | 0.27 |
| | 6 Months | | 0.45 | 0.06 | 0.51 |
| | Initial | Alu - Alu blister | 0.04 | BDL | 0.04 |
| | 3 Months | | 0.19 | 0.07 | 0.26 |
| | 6 Months | | 0.32 | 0.05 | 0.37 |
| **SIL/F1b alternative L-HPC grade** | Initial | White opaque PVC/PVdC - blister | BDL | BDL | BDL |
| | 3 Months | | 0.26 | 0.05 | 0.31 |
| | 6 Months | | 0.39 | BDL | 0.39 |
| | Initial | Alu - Alu blister | BDL | BDL | BDL |
| | 3 Months | | 0.24 | BDL | 0.24 |
| | 6 Months | | 0.35 | 0.05 | 0.40 |
| **SIL/F5** | Initial | White opaque PVC/PVdC - blister | 0.02 | 0.07 | 0.20 |
| | 3 Months | | 0.25 | 0.06 | 0.42 |
| | 6 Months | | 1.26 | 0.25 | 1.70 |
| | Initial | Alu - Alu blister | 0.02 | 0.07 | 0.20 |
| | 3 Months | | 0.23 | 0.10 | 0.47 |
| | 6 Months | | 0.65 | 0.07 | 0.83 |

**Table: Comparison of impurity profiles in Alu-Alu blister at 40°C/75%RH for SIL/F1, SIL/F2 SIL/F5 and prior art WO 2012/147107**

| **Example** | **Stage/Condition** | **Total Impurities (%m/m)** |
|---|---|---|
| **SIL/F1** | Initial | 0.20 |
| | 3 Months | 0.35 |
| **SIL/F2, Commercial-001** | Initial | 0.04 |
| | 3 Months | 0.26 |
| | 6 Months | 0.37 |
| **SIL/F1b, Alternative L-HPC grade** | Initial | BDL |
| | 3 Months | 0.24 |
| **SIL/F5** | Initial | 0.20 |
| | 3 Months | 0.47 |
| **Example-28 of** WO 2012/147107 | Initial | 0.32 |
| | 3 Months | 0.50 |
| **Example-29 of** WO 2012/147107 | Initial | 0.31 |
| | 3 Months | 0.48 |

**Table: ICH stability under accelerated conditions (40°C/75%RH) in opaque PVC/PVdC blister**

| **Batch No.** | **Stage/Condition** | **Impurity (%m/m)** | | |
|---|---|---|---|---|
| | | **Impurity-A** | **Max. Unknown** | **Total impurities** |
| **SIL/F2, Commercial-001** | Initial | 0.04 | BDL | 0.04 |
| | 3 Months | 0.27 | BDL | 0.27 |
| | 6 Months | 0.45 | 0.06 | 0.51 |
| **SIL/F2, Commercial-002** | Initial | 0.04 | BDL | 0.04 |
| | 3 Months | 0.22 | BDL | 0.22 |
| | 6 Months | 0.39 | 0.05 | 0.43 |
| **SIL/F2, Commercial-003** | Initial | 0.03 | 0.05 | 0.08 |
| | 3 Months | 0.22 | BDL | 0.22 |
| | 6 Months | 0.41 | 0.05 | 0.46 |
| **SIL/F2, Commercial-004** | Initial | 0.05 | 0.07 | 0.12 |
| | 3 Months | 0.26 | BDL | 0.26 |
| | 6 Months | 0.43 | 0.06 | 0.49 |
| **SIL/F2, Commercial-005** | Initial | 0.04 | 0.07 | 0.11 |
| | 3 Months | 0.20 | BDL | 0.20 |
| | 6 Months | 0.37 | BDL | 0.37 |
| **SIL/F2, Commercial-006** | Initial | 0.03 | 0.07 | 0.10 |
| | 3 Months | 0.19 | BDL | 0.19 |
| | 6 Months | 0.35 | 0.05 | 0.40 |

**Table: ICH stability under accelerated conditions (40°C/75%RH) in PVC/PE/PVdC blister**

| **Batch No.** | **Stage/Condition** | **Impurity (%m/m)** | | |
|---|---|---|---|---|
| | | **Impurity-A** | **Max. Unknown** | **Total impurities** |
| **SIL/F2, Commercial-001** | Initial | 0.04 | BDL | 0.04 |
| | 3 Months | 0.25 | 0.06 | 0.31 |
| | 6 Months | 0.43 | 0.06 | 0.49 |
| **SIL/F2, Commercial-002** | Initial | 0.04 | BDL | 0.04 |
| | 3 Months | 0.19 | BDL | 0.19 |
| | 6 Months | 0.34 | 0.05 | 0.39 |
| **SIL/F2, Commercial-003** | Initial | 0.03 | 0.05 | 0.08 |
| | 3 Months | 0.19 | BDL | 0.19 |
| | 6 Months | 0.34 | 0.05 | 0.39 |
| **SIL/F2, Commercial-004** | Initial | 0.05 | 0.07 | 0.12 |
| | 3 Months | 0.23 | 0.06 | 0.29 |
| | 6 Months | 0.40 | 0.05 | 0.50 |
| **SIL/F2, Commercial-005** | Initial | 0.04 | 0.07 | 0.11 |
| | 3 Months | 0.18 | BDL | 0.18 |
| | 6 Months | 0.33 | BDL | 0.33 |
| **SIL/F2, Commercial-006** | Initial | 0.03 | 0.07 | 0.10 |
| | 3 Months | 0.18 | 0.05 | 0.23 |
| | 6 Months | 0.30 | 0.05 | 0.35 |

**Table: ICH stability under accelerated conditions (40°C/75%RH) in Alu-Alu blister**

| **Batch No.** | **Stage/Condition** | **Impurity (%m/m)** | | |
|---|---|---|---|---|
| | | **Impurity-A** | **Max. Unknown** | **Total impurities** |
| **SIL/F2, Commercial-001** | Initial | 0.04 | BDL | 0.04 |
| | 3 Months | 0.19 | 0.07 | 0.26 |
| | 6 Months | 0.32 | 0.05 | 0.37 |
| **SIL/F2, Commercial-002** | Initial | 0.04 | BDL | 0.04 |
| | 3 Months | 0.16 | 0.05 | 0.21 |
| | 6 Months | 0.25 | BDL | 0.25 |
| **SIL/F2, Commercial-003** | Initial | 0.03 | 0.05 | 0.08 |
| | 3 Months | 0.17 | 0.05 | 0.22 |
| | 6 Months | 0.28 | BDL | 0.28 |
| **SIL/F2, Commercial-004** | Initial | 0.05 | 0.07 | 0.12 |
| | 3 Months | 0.17 | 0.07 | 0.24 |
| | 6 Months | 0.30 | 0.07 | 0.37 |
| **SIL/F2, Commercial-005** | Initial | 0.04 | 0.07 | 0.11 |
| | 3 Months | 0.15 | 0.05 | 0.20 |
| | 6 Months | 0.24 | 0.05 | 0.29 |
| **SIL/F2, Commercial-006** | Initial | 0.03 | 0.07 | 0.10 |
| | 3 Months | 0.14 | 0.05 | 0.19 |
| | 6 Months | 0.24 | 0.05 | 0.29 |

**Table: ICH stability under long-term conditions (25°C/60%RH) in opaque PVC/PVdC blister**

| **Batch No.** | **Stage/ Condition** | **Impurity (%m/m)** | | |
|---|---|---|---|---|
| | | **Impurity-A** | **Max. Unknown** | **Total impurities** |
| **SIL/F2, Commercial-001** | Initial | 0.04 | BDL | 0.04 |
| | 3 Months | 0.06 | BDL | 0.06 |
| | 6 Months | 0.08 | BDL | 0.08 |
| **SIL/F2, Commercial-002** | Initial | 0.04 | BDL | 0.04 |
| | 3 Months | 0.05 | BDL | 0.05 |
| | 6 Months | 0.07 | BDL | 0.07 |
| **SIL/F2, Commercial-003** | Initial | 0.03 | 0.05 | 0.08 |
| | 3 Months | 0.05 | BDL | 0.05 |
| | 6 Months | 0.07 | BDL | 0.07 |
| **SIL/F2, Commercial-004** | Initial | 0.05 | 0.07 | 0.12 |
| | 3 Months | 0.06 | BDL | 0.06 |
| | 6 Months | 0.08 | BDL | 0.08 |
| **SIL/F2, Commercial-005** | Initial | 0.04 | 0.07 | 0.11 |
| | 3 Months | 0.05 | BDL | 0.05 |
| | 6 Months | 0.07 | BDL | 0.07 |
| **SIL/F2, Commercial-006** | Initial | 0.03 | 0.07 | 0.10 |
| | 3 Months | 0.05 | BDL | 0.05 |
| | 6 Months | 0.07 | BDL | 0.07 |

**Table: ICH stability under long-term conditions (25°C/60%RH) in PVC/PE/PVdC blister**

| **Batch No.** | **Stage/Condition** | **Impurity (%m/m)** | | |
|---|---|---|---|---|
| | | **Impurity-A** | **Max. Unknown** | **Total impurities** |
| **SIL/F2, Commercial-001** | Initial | 0.04 | BDL | 0.04 |
| | 3 Months | 0.06 | BDL | 0.06 |
| | 6 Months | 0.08 | BDL | 0.08 |
| **SIL/F2, Commercial-002** | Initial | 0.04 | BDL | 0.04 |
| | 3 Months | 0.05 | BDL | 0.05 |
| | 6 Months | 0.07 | BDL | 0.07 |
| **SIL/F2, Commercial-003** | Initial | 0.03 | 0.05 | 0.08 |
| | 3 Months | 0.05 | BDL | 0.05 |
| | 6 Months | 0.07 | BDL | 0.07 |
| **SIL/F2, Commercial-004** | Initial | 0.05 | 0.07 | 0.12 |
| | 3 Months | 0.05 | BDL | 0.05 |
| | 6 Months | 0.07 | BDL | 0.07 |
| **SIL/F2, Commercial-005** | Initial | 0.04 | 0.07 | 0.11 |
| | 3 Months | 0.05 | BDL | 0.05 |
| | 6 Months | 0.06 | BDL | 0.06 |
| **SIL/F2, Commercial-006** | Initial | 0.03 | 0.07 | 0.10 |
| | 3 Months | 0.05 | BDL | 0.05 |
| | 6 Months | 0.06 | BDL | 0.06 |

**Table: ICH stability under long-term conditions (25°C/60%RH) in Alu-Alu blister**

| **Batch No.** | **Stage/ Condition** | **Impurity (%m/m)** | | |
|---|---|---|---|---|
| | | **Impurity-A** | **Max. Unknown** | **Total impurities** |
| **SIL/F2, Commercial-001** | Initial | 0.04 | BDL | 0.04 |
| | 3 Months | 0.06 | BDL | 0.06 |
| | 6 Months | 0.07 | BDL | 0.07 |
| **SIL/F2, Commercial-002** | Initial | 0.04 | BDL | 0.04 |
| | 3 Months | 0.05 | BDL | 0.05 |
| | 6 Months | 0.06 | BDL | 0.06 |
| **SIL/F2, Commercial-003** | Initial | 0.03 | 0.05 | 0.08 |
| | 3 Months | 0.05 | BDL | 0.05 |
| | 6 Months | 0.06 | BDL | 0.06 |
| **SIL/F2, Commercial-004** | Initial | 0.05 | 0.07 | 0.12 |
| | 3 Months | 0.05 | BDL | 0.05 |
| | 6 Months | 0.07 | BDL | 0.07 |
| **SIL/F2, Commercial-005** | Initial | 0.04 | 0.07 | 0.11 |
| | 3 Months | 0.05 | BDL | 0.05 |
| | 6 Months | 0.06 | BDL | 0.06 |
| **SIL/F2, Commercial-006** | Initial | 0.03 | 0.07 | 0.10 |
| | 3 Months | 0.05 | BDL | 0.05 |
| | 6 Months | 0.06 | BDL | 0.06 |

As shown above, comparative examples were carried out using the formulations disclosed in prior art WO 2012/147107. In particular, a comparison of impurity profiles in SIL/F1, SIL/F2, SIL/F5 and Examples 28 and 29 of WO 2012/147107 after storage in Alu-Alu blisters at 40°C/75%RH was carried out. Both initial values of total impurities, in addition to total impurities after 3 months storage, show that the formulations disclosed in Examples 28 and 29 of WO 2012/147107 are associated with higher levels of impurities in comparison to the inventive examples SIL/F1, SIL/F2 and SIL/F5.

Furthermore, the data presented above demonstrates excellent stability profiles for the SIL/F1, SIL/F2, SIL/F5 formulations, including commercial formulations of SIL/F2, in a range of packaging blisters and over 3 or 6 months. Additionally, the dissolution properties of the formulations after accelerated and long-term studies show no detrimental change and lie within required specifications.

According to the SmPC of the reference product Urorec, it has to be stored under 25°C in order to maintain sufficient stability. This required labeling of the reference product is based on accelerated and intermediate storage data, the product was stable only under long term conditions. However, the formulations of the present invention, in particular the commercial batches disclosed above, do not require any cooling and can be stored also in warmer conditions, for example in warmer countries at ambient conditions. When assessing the stability data for comparative example SIL/C2, it has 1.51 % of impurity A after a 6-month accelerated study. Per ICH guidelines, the level of the known impurity should be 0.5% or less. In contrast to the reference product, our product with the optimized composition SIL/F2 is in line with the guidance. All our commercial batches in the same package are showing 0.5% or less, refer the table "Table: ICH stability under accelerated conditions (40°C/75%RH) in opaque PVC/PVdC blister".

In conclusion, the capsules packed in Alu-Alu, Opaque PVC/PE/PVdC-Alu and Opaque PVC/PVdC-Alu blisters are stable under accelerated as well as under long term conditions. No variations in the physico-chemical parameters and no decline in assay or dissolution properties was evident. No significant change is observed in the quality of Silodosin capsules.

From our stability study it can be inferred that Silodosin capsules are stable for at least 6 months using the packing material Alu-Alu blister, Opaque PVC/PE/PVdC-Alu blisters and Opaque PVC/PVdC-Alu blisters and show improvements over the reference product.

### Part D-2: Polymorphic Stability:

The assessments of chemical stability (impurity profile; refer to table 2 in part D) shows that both SIL/F1 and SIL/F5 show reduced amounts of Impurity-A and reduced total impurities after storage in white opaque PVC/ PVdC-blister after 6 months at 40°C/75%RH when compared to a commercial product.

Using the same conditions, the stability of the polymorphic form of silodosin was tested using X-ray diffraction (XRD) analysis. The API employed in this example (SIL/F1) is polymorphic form Beta. The API source is from South Korea.

### XRD Method:

A summary of XRD method parameters for polymorphic form identification is described below.

| | |
|---|---|
| Make/Model: | PANalytical/EMPYREAN |
| X-Ray Tube: | Copper Kα1, 1.5406 Å |
| Detector: | Pixcel |
| Kβ Filter: | Nickel |
| Start Position (°2θ): | 2.0 |
| End Position (°2θ): | 40.0 |
| Step Size (°2θ): | 0.013 |
| Step Time (sec): | 290.445 |
| Divergence Slit: | Fixed, 0.5° |
| Anti-Scattering Slit: | P8 mm |
| Current: | 40 mA |
| Voltage: | 45 kV |
| Scan Type: | Continuous |
| Spinning: | Yes |
| Scan Time: | 1 Hr |
| Sample quantity: | 350 mg (approx.) |
| Sample holder diameter: 16 mm | |

Comparisons in XRD patterns were carried out between the Beta form employed in the examples at time points 0 and at 6 months after storage (under the conditions mentioned above), Beta form controls, Alpha form controls and a placebo. Only the Beta form is observed in capsules initially (time point 0) and after 6 months storage. No Alpha form is observed in either Beta form controls, or at time points 0 or 6. Hence, no conversion of Beta form to Alpha Form was observed during stability testing at 40°C/75% RH for 6 months.

### Part E

### Blend Uniformity and Content Uniformity of Capsules:

Blend uniformity and content uniformity of capsules produced according to the invention were conducted. The formulation according to SIL/F2 (Example 2) was employed. Two batches were prepared according to Example 2 on a pilot and commercial scale using 2 different sources of active pharmaceutical ingredient (API; silodosin), obtained from China (CN-API) and India (IN-API). No difficulties in filling (such as sticking) were observed in any batches employing SIL/F2.

**Table: Batches prepared for assessment of blend and content uniformity**

| **S. No.** | **Ingredients** | **SIL/F2 (CN-API)** | | **SIL/F2 (IN-API)** | |
|---|---|---|---|---|---|
| | | **Common Blend ≈10,000 cap** | | **Common Blend ≈ 45,000 cap** | |
| | | **4 mg** | **8 mg** | **4 mg** | **8 mg** |
| **Batch size** | | **10,000 cap** | **2,000 cap** | **45,000 cap** | **3,500 cap** |
| **Blending & Capsule filling** | | **mg/capsule** | | | |
| 1 | Silodosin | 4.00 | 8.00 | 4.00 | 8.00 |
| 2 | Mannitol (Pearlitol 200 SD) | 97.40 | 194.80 | 97.40 | 194.80 |
| 3 | Low-substituted Hydroxy Propyl Cellulose (L-HPC LH-21) | 45.00 | 90.00 | 45.00 | 90.00 |
| 4 | Sodium lauryl sulfate | 1.80 | 3.60 | 1.80 | 3.60 |
| 5 | Magnesium stearate | 1.80 | 3.60 | 1.80 | 3.60 |
| **Weight of blend** | | **150.00** | **300.00** | **150.00** | **300.00** |
| 6 | EHG capsule (Size "3") | 50 | - | 50 | - |
| **7** | EHG capsule (Size "1") | - | 76 | - | 76 |
| **Weight of capsule** | | **200.00** | **376.00** | **200.00** | **376.00** |

**Table: Results from assessment of blend uniformity employing two sources of API**

| **Blend Uniformity (15 min)** | | |
|---|---|---|
| **Batch no:** | **SIL/F2 (CN-API) ≈10, 000 cap** | **SIL/F2 (IN-API) =45, 000 cap** |
| **Strength** | **4 mg** | |

| Location No. | % Assay | % Assay |
|---|---|---|
| 1 | 104.0 | 101.0 |
| 2 | 97.0 | 100.6 |
| 3 | 99.8 | 100.7 |
| 4 | 97.9 | 98.4 |
| 5 | 98.2 | 104.20 |
| 6 | 100.2 | 99.9 |
| 7 | 105.0 | 103.6 |
| 8 | 103.0 | 98.90 |
| 9 | 98.6 | 108.0 |
| 10 | 114.8 | 99.90 |
| **Mean** | **101.8** | **101.50** |
| Min | 97.0 | 98.40 |
| Max | 114.8 | 108.0 |
| **% RSD** | **5.20** | **2.89** |
| **Blend Assay** | **100.3** | **99.10** |

**Table: Results from assessment of content uniformity employing two sources of API**

| **Content Uniformity of Capsules** | | | | |
|---|---|---|---|---|
| **Batch no:** | **SIL/F2 (CN-API)** | | **SIL/F2 (IN-API)** | |
| **Strength** | **4 mg** | **8 mg** | **4 mg** | **8 mg** |
| Capsule No. | % Assay | | | |
| 1 | 102.0 | 101.1 | 99.1 | 96.6 |
| 2 | 95.7 | 99.9 | 99.9 | 98.0 |
| 3 | 99.0 | 103.8 | 103.9 | 99.7 |
| 4 | 99.2 | 102.5 | 100.9 | 99.1 |
| 5 | 101.1 | 100.2 | 101.1 | 102.9 |
| 6 | 101.5 | 95.4 | 101.6 | 100.8 |
| 7 | 101.3 | 101.5 | 106.1 | 100.5 |
| 8 | 103.7 | 102.1 | 97.20 | 101.3 |
| 9 | 103.1 | 104.7 | 103.1 | 97.9 |
| 10 | 106.5 | 102.8 | 101.5 | 99.7 |
| **Mean** | **101.3** | **101.4** | **101.5** | **99.7** |
| Min | 95.7 | 95.4 | 97.2 | 96.6 |
| Max | 106.5 | 104.7 | 106.1 | 102.9 |
| **% RSD** | **2.90** | **2.56** | **2.48** | **1.86** |
| **AV value** | **7.1** | **6.2** | **6.1** | **4.4** |
| **Capsule Assay** | **100.1** | **99.9** | **102.1** | **100.6** |

As can be observed from the data presented above, the content uniformity (both for the blend and for the capsules) is found to be within the specification limits according to European Pharmacopeia 2.9.40. To ensure the consistency of dosage units, each unit in a batch should have an active substance content within a narrow range around the label claim. Dosage units are defined as dosage forms containing a single dose or a part of a dose of an active substance in each dosage unit. The term "Uniformity of dosage unit" is defined as the degree of uniformity in the amount of the active substance among dosage units. Therefore, the requirements of this regulation can be applied to any active substance being comprised in dosage units containing one or more active substances. The uniformity of dosage forms (by content uniformity) was assessed according to European Pharmacopeia 2.9.40. The assay (%) of 10 individual capsules was detected by HPLC analysis according to the following HPLC conditions:

| | |
|---|---|
| Column | : ACE 5 C18 PFP 250x4.6 mm, 5 µm or equivalent |
| Flow rate | : 1.0 mL/min |
| Detection | : UV, 270 nm |
| Injection Volume | : 10 µL |
| Data acquisition time | : 9 minutes |
| Pump mode | : Isocratic |
| Column temperature | : 30° C. |

The accepted maximum allowed acceptance value (AV) L1 = 15.0 was satisfied by all batches tested.

Furthermore, the content uniformity displayed by the formulations described herein appears to be improved compared to the data disclosed by Kissei Pharmaceutical Co. Ltd. during examination of EP 1574215 on March 5, 2010. EP 1574215 relates to capsules prepared by wet granulating the pharmaceutical blend and subsequent addition of SLS and magnesium stearate post-granulation. The "average amount contained" (as described above under "Mean") was described for the technology of EP 1574215 as 99.4%. The study above for SIL/F2 shows a mean value of 99.7-101.5%, clearly satisfying the standard requirements of 95-105%. The "CV Value" (as described above under "RSD") was described for the technology of EP 1574215 as 2.8%. The RSD values described above of 2.48-2.90% for the 4 mg formulation and 1.86-2.56% for the 8 mg formulation show an improvement over the technology of the prior art described in EP 1574215.

### Part F

### Production of Silodosin capsules at commercial batch size:

Three batches each Silodosin 4 mg and 8 mg capsules at a scale of 100,000 capsules (Batch Numbers Commercial-001, Commercial-002 and Commercial-003) and 200,000 capsules (Batch Numbers Commercial-004, Commercial-005, Commercial-006) respectively were manufactured employing the general procedure under Part B above. The composition according to Example 2 (SIL/F2) was employed.

The reference (UROREC® 8 mg Hard Capsules) and test (Silodosin 8 mg Capsules; SIL/F2) products were subjected to physicochemical evaluation and the results are presented in the Table below. Data obtained from the test product is comparable to the reference product.

**Table: Physicochemical evaluation of Reference and Test product**

| | **Reference** | **Test (SIL/F2)** |
|---|---|---|
| Product | UROREC® 8 mg Hard Capsules | Silodosin 8 mg Capsules |
| Lot No./B. No. | 16466 | Commercial-006 |
| Exp. Date | 09-2019 | - |
| Batch size | NA | 200,000 Capsules |
| Description | White, opaque, hard gelatin capsule containing white powder. | White, opaque, hard gelatin capsule containing off-white powder. |
| Average net content (mg) | 350.6 | 304.1 |
| Capsule size | Size "0" | Size "1" |
| Locked length (mm) | 21.37 - 21.45 | 19.32 - 19.41 |
| Disintegration time (min) | 4 min 40 sec | 5 min 11 sec |
| Assay (% of Label claim) | 98.0 | 110.5 |
| Related substances (%m/m): | | |
| Impurity-A | 0.05 | 0.03 |
| Impurity-B | Below disregard limit | Not detected |
| Unspecified impurity | Below disregard limit | 0.07 |
| Total impurities excluding Impurity-A | 0.05 | 0.07 |
| Uniformity of dosage units: Acceptance value | 12.0 | 2.96 |

The content uniformity was found to be within the specification limits according to European Pharmacopeia 2.9.40. The accepted maximum allowed acceptance value (AV) L1 = 15.0 was satisfied by the commercial scale batches tested. As mentioned in the above table, the AV value determined for batch Commercial-006 (test product) was 2.96, showing an improvement over the reference product (AV = 12.0).

Similarly, dissolution of the reference and the test product were examined in various release dissolution media as described in the methodology above under Part C. In short, 900 mL dissolution medium was employed using a paddle with sinker at 50 rpm. The data obtained from the test product is comparable to the reference product.

**Table: Dissolution of Silodosin 4 mg Capsules (Reference and Test) in dissolution medium**

| **Drug dissolved in phosphate buffer pH 6.8, 1500 ml, paddle with sinker at 25 rpm** | | | | |
|---|---|---|---|---|
| **Time (min)** | **Urorec® 4 mg (Lot: SE6B65)** | **Commercial-001** | **Commercial-002** | **Commercial-003** |
| | Drug dissolved (%) | Drug dissolved (%) | Drug dissolved (%) | Drug dissolved (%) |
| 5 | 40 | 41 | 45 | 47 |
| 10 | 66 | 67 | 66 | 69 |
| 15 | 71 | 76 | 77 | 73 |
| 20 | 74 | 80 | 78 | 75 |
| 30 | 78 | 84 | 82 | 79 |
| 45 | 83 | 89 | 87 | 84 |
| 60 | 86 | 91 | 90 | 86 |
| Recovery | 100 | 104 | 103 | 100 |

**Table: Dissolution of Silodosin 8 mg Capsules (Reference and Test) in dissolution medium**

| **Drug dissolved in phosphate buffer pH 6.8, 1500 ml, paddle with sinker at 25 rpm** | | | | |
|---|---|---|---|---|
| **Time (min)** | **Urorec® 8 mg (Lot: 16466)** | **Commercial-004** | **Commercial-005** | **Commercial-006** |
| | **Drug dissolved (%)** | **Drug dissolved (%)** | **Drug dissolved (%)** | **Drug dissolved (%)** |
| 5 | 36 | 38 | 42 | 43 |
| 10 | 54 | 58 | 57 | 58 |
| 15 | 62 | 69 | 66 | 64 |
| 20 | 66 | 74 | 67 | 69 |
| 30 | 72 | 79 | 71 | 73 |
| 45 | 78 | 82 | 74 | 76 |
| 60 | 80 | 83 | 77 | 78 |
| Recovery | 100 | 101 | 101 | 100 |

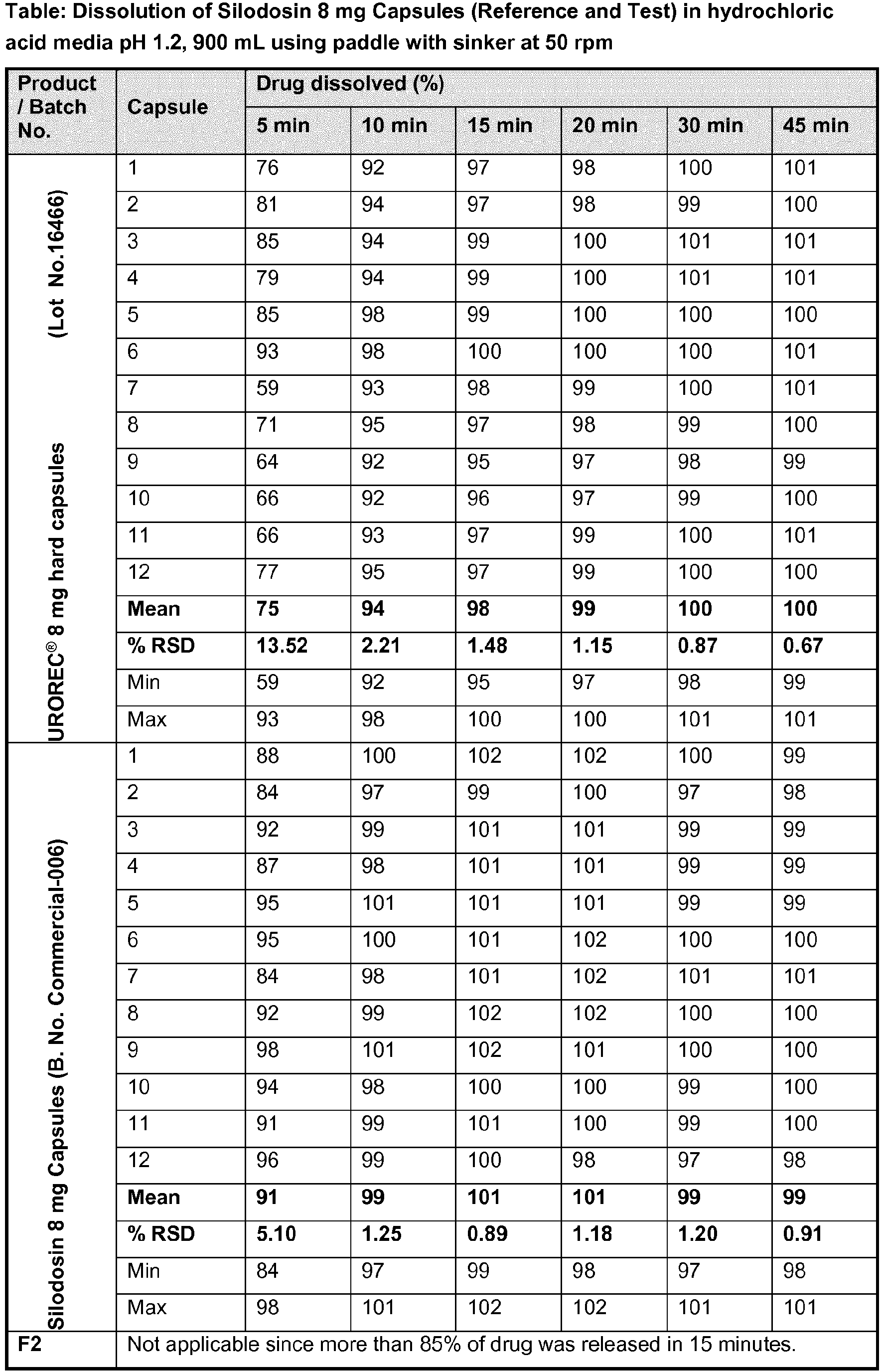

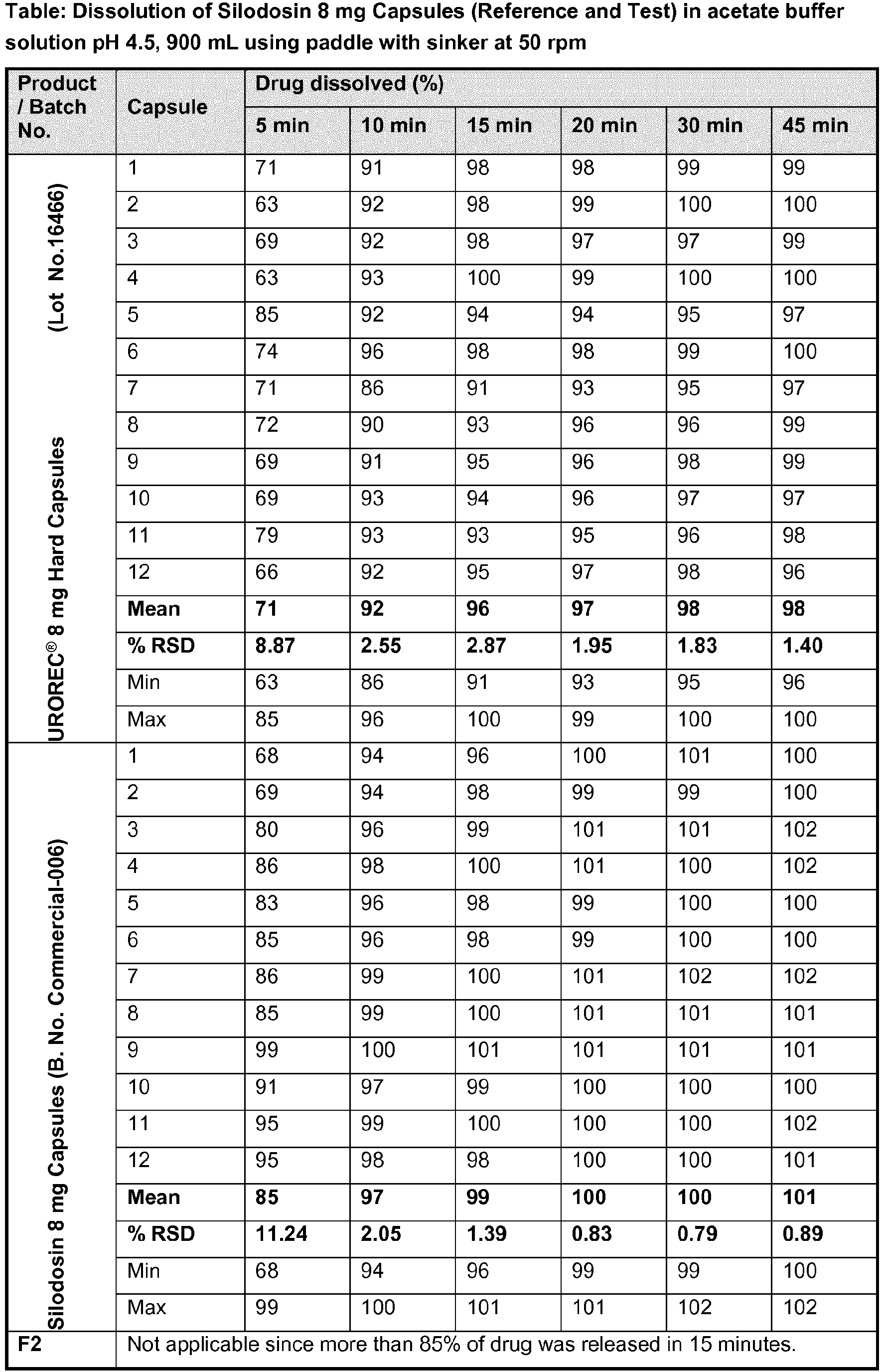

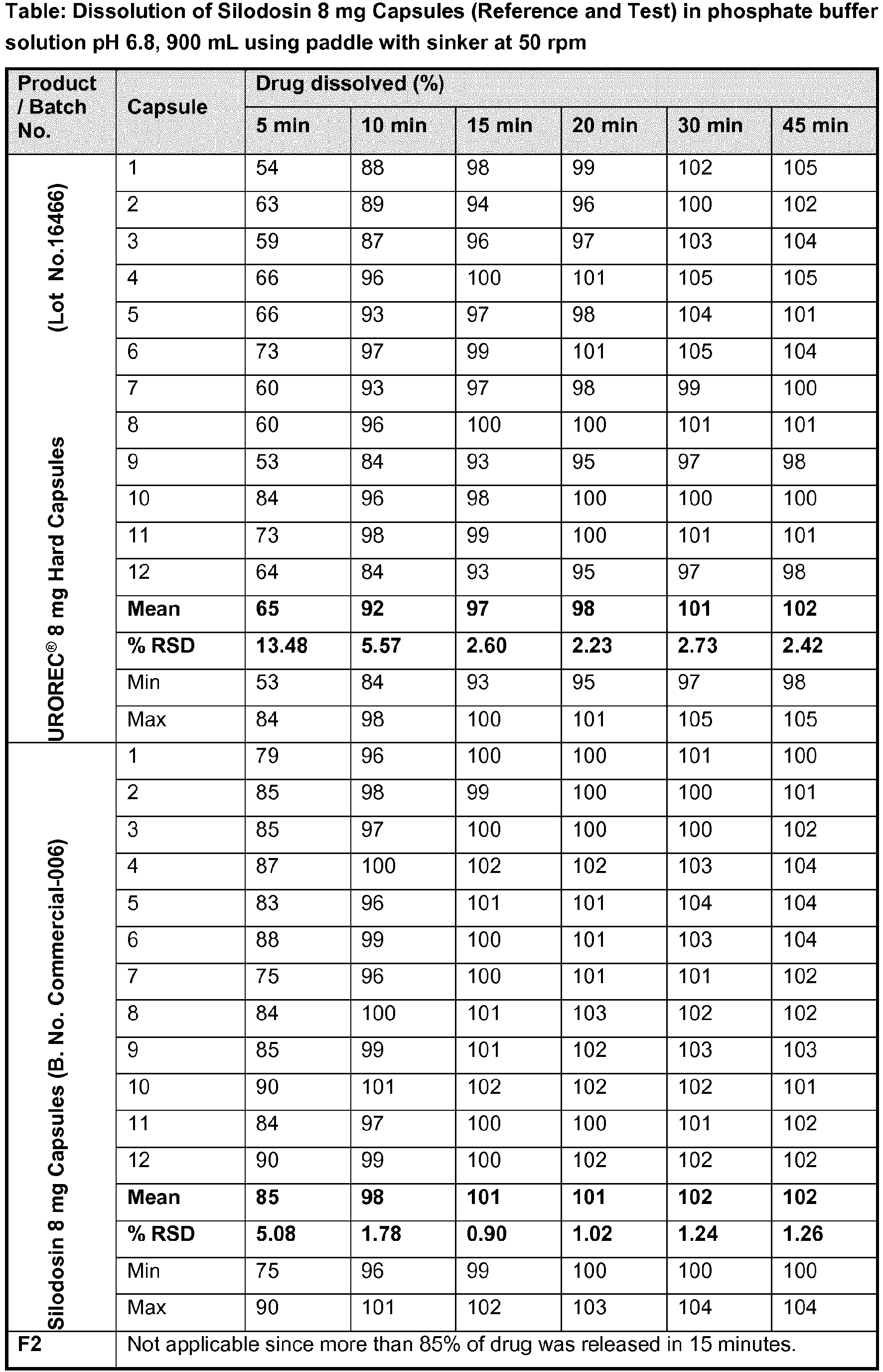

The dissolution in release medium is found to be similar with more than 85% drug release in 15 min from both test and reference products. In addition, the dissolutions in acetate buffer solution pH 4.5 and phosphate buffer solution pH 6.8 are found to be similar to the reference product. Based on above evaluation, it is expected that the in vivo behavior of test product would remain similar to reference product.

## Claims

1. A pharmaceutical composition in the form of a capsule, comprising a powder mixture that comprises a) silodosin, b) a surfactant, c) a lubricant, and d) low-substituted hydroxypropyl cellulose (L-HPC) as a disintegrant in an amount of 16 wt% or more based on the total weight of all components of the mixture, and optionally e) a diluent, wherein said composition is prepared by dry mixing or blending the silodosin, disintegrant, surfactant, lubricant, and optional diluent, in a single powder mixture or blend, followed by direct filling into a capsule.

2. The pharmaceutical composition according to claim 1, wherein the diluent is D-mannitol.

3. The pharmaceutical composition according to claim 1, wherein the diluent is selected from sorbitol and/or xylitol.

4. The pharmaceutical composition according to any one of the preceding claims, wherein the surfactant is sodium lauryl sulfate (SLS).

5. The pharmaceutical composition according to any one of the preceding claims, wherein the lubricant is magnesium stearate.

6. The pharmaceutical composition according to any one of the preceding claims, wherein the diluent is D-mannitol and/or sorbitol, the disintegrant is L-HPC, the surfactant is SLS and the lubricant is magnesium stearate.

7. The pharmaceutical composition according to claim 1, wherein the disintegrant is present in an amount of 16-96 wt%, preferably 18-50 wt%, more preferably 20-46%, based on the total weight of all components of the mixture.

8. The pharmaceutical composition according to claim 1, wherein a) the silodosin is present in an amount of 1-4 wt%, preferably 2-4 wt%, b) the surfactant is present in an amount of 0.1-5 wt%, preferably 0.5-2 wt%, c) the lubricant is present in an amount of 0.1-5 wt%, preferably 0.5-2 wt%, d) the disintegrant is present in an amount 16-96 wt%, preferably 18-50 wt% and e) the diluent is optionally present in an amount of 10-80 wt%, preferably 40-80 wt%, based on the total weight of all components of the mixture.

9. The pharmaceutical composition according to claim 1, wherein the diluent is D-mannitol and the disintegrant is L-HPC, and wherein the ratio of D-mannitol to L-HPC in the mixture is from 6:1 to 1:1, preferably from 4:1 to 1:1.

10. The pharmaceutical composition according to claim 1, wherein a) the silodosin is present in an amount of 1-4 wt%, preferably 2-4 wt%, b) the surfactant is SLS and is present in an amount of 0.1-5 wt%, preferably 0.5-2 wt%, c) the lubricant is magnesium stearate and is present in an amount of 0.1-5 wt%, preferably 0.5-2 wt%, d) the disintegrant is L-HPC and is present in an amount 16-50 wt%, and e) the diluent is D-mannitol and is present in an amount of 40-80 wt%, based on the total weight of all components of the mixture.

11. The pharmaceutical composition according to any one of the preceding claims, wherein the capsule is a light shielding capsule, preferably comprising titanium dioxide.

12. The pharmaceutical composition according to any one of the preceding claims for use as a medicament in the treatment of dysuria and/or benign prostatic hyperplasia.

13. Method of preparing a pharmaceutical composition in the form of a capsule, said capsule comprising a powder mixture of silodosin, a diluent, low-substituted hydroxypropyl cellulose (L-HPC) as a disintegrant in an amount of 16%wt or more based on the total weight of all components of the mixture, a surfactant and a lubricant, wherein said methods comprises dry mixing or blending the silodosin, diluent, disintegrant, surfactant and lubricant in a single powder mixture or blend, optionally sifting the mixture or blend, followed by direct filling into a capsule, wherein the method does not comprise granulation.

## Patentansprüche

1. Pharmazeutische Zusammensetzung in Form einer Kapsel, umfassend eine Pulvermischung, die a) Silodosin, b) ein Tensid, c) ein Schmiermittel und d) niedrigsubstituierte Hydroxypropylcellulose (L-HPC) als Sprengmittel in einer Menge von 16 Gew.-% oder mehr, bezogen auf das Gesamtgewicht aller Komponenten der Mischung, und gegebenenfalls e) einen Füllstoff umfasst, wobei die Zusammensetzung hergestellt wird, indem Silodosin, das Sprengmittel, das Tensid, das Schmiermittel und der optionale Füllstoff in einer einzelnen Pulvermischung trocken gemischt werden, gefolgt von einer Direktabfüllung in eine Kapsel.

2. Pharmazeutische Zusammensetzung nach Anspruch 1, wobei der Füllstoff D-Mannitol ist.

3. Pharmazeutische Zusammensetzung nach Anspruch 1, wobei der Füllstoff aus Sorbitol und/oder Xylitol ausgewählt ist.

4. Pharmazeutische Zusammensetzung nach einem der vorhergehenden Ansprüche, wobei das Tensid Natriumlaurylsulfat (SLS) ist.

5. Pharmazeutische Zusammensetzung nach einem der vorhergehenden Ansprüche, wobei das Schmiermittel Magnesiumstearat ist.

6. Pharmazeutische Zusammensetzung nach einem der vorhergehenden Ansprüche, wobei der Füllstoff D-Mannitol und/oder Sorbitol ist, das Sprengmittel L-HPC ist, das Tensid SLS ist und das Schmiermittel Magnesiumstearat ist.

7. Pharmazeutische Zusammensetzung nach Anspruch 1, wobei das Sprengmittel in einer Menge von 16 bis 96 Gew.-% vorliegt, vorzugsweise 18 bis 50 Gew.-%, bevorzugter 20 bis 46% Gew.-%, bezogen auf das Gesamtgewicht aller Komponenten der Mischung.

8. Pharmazeutische Zusammensetzung nach Anspruch 1, wobei a) das Silodosin in einer Menge von 1 bis 4 Gew.-%, vorzugsweise 2 bis 4 Gew.-%, b) das Tensid in einer Menge von 0,1 bis 5 Gew.-%, vorzugsweise 0,5 bis 2 Gew.-%, c) das Schmiermittel in einer Menge von 0,1 bis 5 Gew.-%, vorzugsweise 0,5 bis 2 Gew.-%, d) das Sprengmittel in einer Menge von 16 bis 96 Gew.-%, vorzugsweise 18 bis 50 Gew.-% und gegebenenfalls e) der Füllstoff in einer Menge von 10 bis 80 Gew.-%, vorzugsweise 40 bis 80 Gew.-%, vorliegen, jeweils bezogen auf das Gesamtgewicht aller Komponenten der Mischung.

9. Pharmazeutische Zusammensetzung nach Anspruch 1, wobei der Füllstoff D-Mannitol und das Sprengmittel L-HPC ist und worin das Verhältnis von D-Mannitol zu L-HPC in der Mischung 6:1 bis 1:1 beträgt, vorzugsweise 4:1 bis 1:1.

10. Pharmazeutische Zusammensetzung nach Anspruch 1, wobei a) das Silodosin in einer Menge von 1 bis 4 Gew.-% vorliegt, vorzugsweise 2 bis 4 Gew.-%, b) das Tensid SLS ist und in einer Menge von 0,1 bis 5 Gew.-% vorliegt, vorzugsweise 0,5 bis 2 Gew.-%, c) das Schmiermittel Magnesiumstearat ist und in einer Menge von 0,1 bis 5 Gew.-% vorliegt, vorzugsweise 0,5 bis 2 Gew.-%, d) das Sprengmittel L-HPC ist und in einer Menge von 16 bis 50 Gew.-% vorliegt und e) der Füllstoff D-Mannitol ist und in einer Menge von 40 bis 80 Gew.-% vorliegt, jeweils bezogen auf das Gesamtgewicht aller Komponenten der Mischung.

11. Pharmazeutische Zusammensetzung nach einem der vorhergehenden Ansprüche, wobei die Kapsel eine vor Licht schützende Kapsel ist, die vorzugsweise Titandioxid umfasst.

12. Pharmazeutische Zusammensetzung nach einem der vorhergehenden Ansprüche zur Verwendung als Medikament bei der Behandlung von Dysurie und/oder benigner Prostatahyperplasie.

13. Verfahren zur Herstellung einer pharmazeutischen Zusammensetzung in Form einer Kapsel, wobei die Kapsel eine Pulvermischung aus Silodosin, einem Füllstoff, niedrigsubstituierter Hydroxypropylcellulose als Sprengmittel in einer Menge von 16 Gew.-% oder mehr, bezogen auf das Gesamtgewicht aller Komponenten der Mischung, einem Tensid und einem Schmiermittel umfasst, wobei das Verfahren eine Trockenmischung von Silodosin, dem Füllstoff, dem Sprengmittel, dem Tensid und dem Schmiermittel in einer einzelnen Pulvermischung umfasst, gegebenenfalls Sieben der Mischung, gefolgt von einer Direktabfüllung in eine Kapsel, wobei das Verfahren keine Granulierung umfasst.

## Revendications

1. Composition pharmaceutique sous la forme d'une capsule, comprenant un mélange de poudres qui comprend a) de la silodosine, b) un tensioactif, c) un lubrifiant, et d) de l'hydroxypropylcellulose (L-HPC) faiblement substituée comme désintégrant en une quantité de 16 % en poids ou plus sur la base du poids total de tous les composants du mélange, et éventuellement e) un diluant, dans laquelle ladite composition est préparée par mélange à sec de la silodosine, d'un désintégrant, d'un tensioactif, d'un lubrifiant et éventuellement d'un diluant, dans un seul mélange ou mélange en poudre, suivi du remplissage direct dans une capsule.

2. Composition pharmaceutique selon la revendication 1, dans laquelle le diluant est le D-mannitol.

3. Composition pharmaceutique selon la revendication 1, dans laquelle le diluant est choisi parmi le sorbitol et/ou le xylitol.

4. Composition pharmaceutique selon l'une quelconque des revendications précédentes, dans laquelle le tensioactif est du laurylsulfate de sodium (SLS).

5. Composition pharmaceutique selon l'une quelconque des revendications précédentes, dans laquelle le lubrifiant est du stéarate de magnésium.

6. Composition pharmaceutique selon l'une quelconque des revendications précédentes, dans laquelle le diluant est le D-mannitol et/ou le sorbitol, le désintégrant est le L-HPC, le tensioactif est le SLS et le lubrifiant est le stéarate de magnésium.

7. Composition pharmaceutique selon la revendication 1, dans laquelle le désintégrant est présent en une quantité de 16 à 96 % en poids, de préférence de 18 à 50 % en poids, plus préférentiellement de 20 à 46 % en poids, sur la base du poids total de tous les composants du mélange.

8. Composition pharmaceutique selon la revendication 1, dans laquelle a) la silodosine est présente en une quantité de 1 à 4% en poids, de préférence de 2 à 4% en poids, b) le tensioactif est présent en une quantité de 0,1 à 5% en poids, de préférence de 0,5 à 2% en poids, c) le lubrifiant est présent en une quantité de 0.1-5 % en poids, de préférence 0,5-2 % en poids, d) le désintégrant est présent en une quantité de 16 à 96 % en poids, de préférence de 18 à 50 % en poids, et e) le diluant est éventuellement présent en une quantité de 10 à 80 % en poids, de préférence de 40 à 80 % en poids, sur la base du poids total de tous les composants du mélange.

9. Composition pharmaceutique selon la revendication 1, dans laquelle le diluant est le D-mannitol et le désintégrant est le L-HPC, et dans laquelle le rapport du D-mannitol au L-HPC dans le mélange est de 6:1 à 1:1, de préférence de 4:1 à 1:1.

10. Composition pharmaceutique selon la revendication 1, dans laquelle a) la silodosine est présente en une quantité de 1 à 4 % en poids, de préférence de 2 à 4 % en poids, b) le tensioactif est SLS et est présent en une quantité de 0,1 à 5 % en poids, de préférence de 0,5 à 2 % en poids, c) le lubrifiant est le stéarate de magnésium et est présent en une quantité de 0.1-5 % en poids, de préférence 0,5-2 % en poids, d) le désintégrant est du L-HPC et est présent en une quantité de 16 à 50 % en poids, et e) le diluant est du D-mannitol et est présent en une quantité de 40 à 80 % en poids, par rapport au poids total de tous les composants du mélange.

11. Composition pharmaceutique selon l'une quelconque des revendications précédentes, dans laquelle la capsule est une capsule de protection contre la lumière, comprenant de préférence du dioxyde de titane.

12. Composition pharmaceutique selon l'une quelconque des revendications précédentes pour utilisation comme médicament dans le traitement de la dysurie et/ou de l'hyperplasie bénigne de la prostate.

13. Procédé de préparation d'une composition pharmaceutique sous la forme d'une capsule, ladite capsule comprenant un mélange en poudre de silodosine, un diluant, de l'hydroxypropylcellulose (L-HPC) faiblement substituée comme désintégrant en une quantité de 16 % en poids ou plus sur la base du poids total de tous les composants du mélange, un tensioactif et un lubrifiant, dans laquelle lesdits procédés comprennent le mélange à sec de la silodosine, du diluant, du désintégrant, du tensioactif et du lubrifiant dans un seul mélange ou mélange en poudre, le tamisage facultatif du mélange ou du mélange, suivi du remplissage direct dans une capsule, le procédé ne comprenant pas de granulation.
